# EUROPEAN PATENT SPECIFICATION

(11) **EP 4 018 973 B1**
(45) Date of publication and mention of the grant of the patent: **10.05.2023**
(21) Application number: 21216533.6
(22) Date of filing: 21.12.2021
(51) Int. Cl.: A61F 2/64, A61F 2/70

(54) **PROSTHETIC KNEE JOINT**
KNIEGELENKPROTHESE
PROTHÈSE D'ARTICULATION DU GENOU

(30) Priority: 25.12.2020 JP 2020216172; 26.10.2021 JP 2021174456
(43) Date of publication of application: 29.06.2022
(73) Proprietor: Nabtesco Corporation, Tokyo 102-0093 (JP)
(72) Inventor: Hashimoto, Hiroaki, Tokyo (JP); Saito, Masakazu, Tokyo (JP); Misao, Taishiro, Tokyo (JP); Miyamoto, Hiroto, Tokyo (JP); Oka, Daisuke, Tokyo (JP)
(74) Representative: Grünecker Patent- und Rechtsanwälte PartG mbB

(56) References cited:
- EP-A1- 3 542 762
- WO-A1-2013/148726
- JP-A- 2013 510 605
- JP-A- 2017 006 339

## Description

### TECHNICAL FIELD

The present disclosure relates to a prosthetic knee joint.

### BACKGROUND

Patent Literature 1 discloses a prosthetic device including a thigh shaft, a lower leg shaft, a prosthetic foot, a joint and a connection part. The prosthetic device has a torque sensor provided in the joint to determine the effective knee torque. The lower leg shaft has a resistance for providing resistance against bending and stretching. The values of the bending- and stretching-resistances are controlled based on sensor data and results of evaluating the sensor data.

### RELEVANT REFERENCES

### LIST OF RELEVANT PATENT LITERATURE

Patent Literature 1: Japanese Patent Application Publication No. 2013-510605

### SUMMARY

Here, Patent Literature 1 discloses the energy consumed when the resistance increases, but does not disclose how the energy consumption is related to the control cycle. Therefore, the technology disclosed in Patent Literature 1 may not achieve sufficiently improved energy efficiency. One object of the present disclosure is to provide a prosthetic knee joint with improved energy efficiency.

In one aspect of the present disclosure, a prosthetic knee joint includes a thigh connection part connectable to a socket configured to receive a thigh of a user, a lower leg part coupled to the thigh connection part such that the lower leg part is rotatable around an axis of a knee, an actuator coupled to the thigh connection part and the lower leg part, where the actuator is configured to restrict or assist movement of the thigh connection part, a detector unit for directly or indirectly obtaining information about how the thigh connection part and the lower leg part are relatively positioned, a control unit for controlling driving of the actuator based on a result detected by the detector unit, and an estimating unit for estimating a state of the user's movement based on the result detected by the detector unit. When the estimating unit estimates that the user is not walking, the control unit sets a longer control cycle for the driving of the actuator than when the estimating unit estimates that the user is walking.

In one aspect of the present disclosure, a prosthetic knee joint includes a thigh connection part connectable to a socket configured to receive a thigh of a user, a lower leg part coupled to the thigh connection part such that the lower leg part is rotatable around an axis of a knee, an actuator coupled to the thigh connection part and the lower leg part, where the actuator is configured to restrict or assist movement of the thigh connection part, a detector unit for directly or indirectly obtaining information about how the thigh connection part and the lower leg part are relatively positioned, and a control unit for controlling driving of the actuator based on a result detected by the detector unit. When a knee angle calculated based on the result detected by the detector unit is within a predetermined range, the control unit sets a longer control cycle for the driving of the actuator than when the knee angle is outside the predetermined range.

In one aspect of the present disclosure, a prosthetic knee joint includes a thigh connection part connectable to a socket configured to receive a thigh of a user, a lower leg part coupled to the thigh connection part such that the lower leg part is rotatable around an axis of a knee, a detector unit for detecting a state of the user's movement, and a communicating unit for communicating with an external device. The communicating unit is enabled to communicate with the external device when the detector unit detects that the user's movement is in a predetermined state.

### ADVANTAGEOUS EFFECTS

The present disclosure can successfully provide a prosthetic knee joint with improved energy efficiency.

### BRIEF DESCRIPTION OF THE DRAWINGS

Fig. 1 shows an example configuration of a prosthetic knee joint according to a first embodiment of the invention.
Fig. 2A specifically shows an example configuration of the prosthetic knee joint of the first embodiment and is a schematic sectional view showing the main part of the prosthetic knee joint.
Fig. 2B specifically shows an example configuration of the prosthetic knee joint of the first embodiment, showing a cylinder and a drive mechanism of the prosthetic knee joint.
Fig. 3 is used to illustrate, as an example, the prosthetic knee joint placed in a bicycle free mode.
Fig. 4 shows an example configuration of a prosthetic knee joint according to a second embodiment of the invention.
Fig. 5A shows a first example of a user's movement required to turn on a wireless communication function between the prosthetic knee joint and an external device.
Fig. 5B shows a second example of the user's movement required to turn on the wireless communication function between the prosthetic knee joint and the external device.

### DESCRIPTION OF THE PREFERRED EMBODIMENTS

The following describes embodiments of a prosthetic knee joint according to the invention with reference to the drawings.

### <First Embodiment>

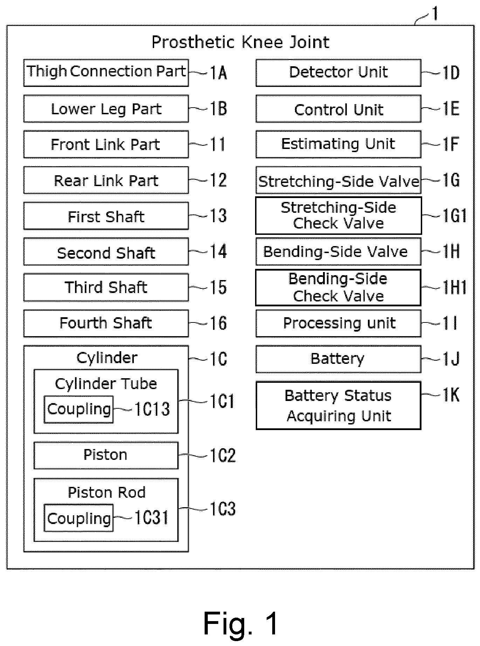

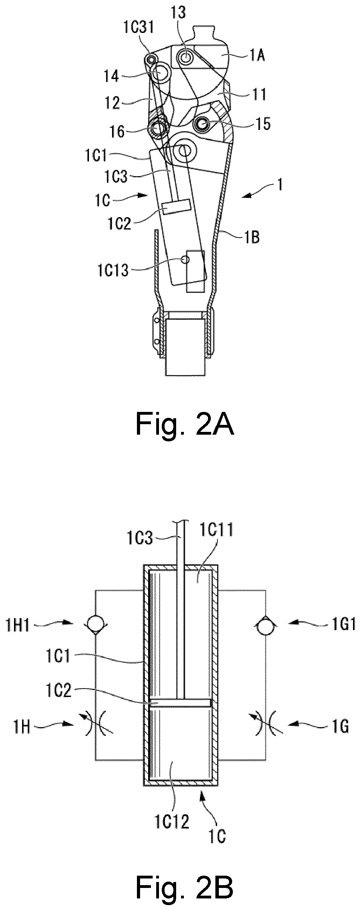

Fig. 1 shows an example configuration of a prosthetic knee joint 1 according to a first embodiment of the invention. Figs. 2A and 2B specifically show an example configuration of the prosthetic knee joint 1. More specifically, Fig. 2A schematically shows an example section of the main part of the prosthetic knee joint 1. Fig. 2B shows, as an example, a cylinder 1C and a drive mechanism of the prosthetic knee joint 1.

As shown in Figs. 1, 2A and 2B, the prosthetic knee joint 1 includes a thigh connection part 1A, a lower leg part 1B, a front link part 11, a rear link part 12, a first shaft 13, a second shaft 14, a third shaft 15, a fourth shaft 16, a cylinder 1C acting as an actuator, and a detector unit 1D. The actuator restricts or assists the movement of the thigh connection part 1A (in detail, the rotational movement of the thigh connection part 1A relative to the lower leg part 1B), as will be described below.

The thigh connection part 1A is connectable to a socket (e.g., see Japanese Patent Application Publication No. 2017-6339 ("the '339 Publication"), Fig. 1) configured to receive the thigh of a user (the wearer of the prosthetic knee joint 1). The lower leg part 1B is coupled to the thigh connection part 1A such that the lower leg part 1B is rotatable around the axis of the knee. In detail, the front link part 11 is coupled to the thigh connection part 1A such that the front link part 11 is rotatable around the first shaft 13. The rear link part 12 is coupled to the thigh connection part 1A such that the rear link part 12 is rotatable around the second shaft 14. The front link part 11 is coupled to the low leg part 1B such that the front link part 11 is rotatable around the third shaft 15. The rear link part 12 is coupled to the low leg part 1B such that the rear link part 12 is rotatable around the fourth shaft 16. In other words, the thigh connection part 1A, the lower leg part 1B, the front link part 11, and the rear link part 12 constitute a four-section link. This means that the lower leg part 1B is rotatable around the axis of the knee (an imaginary axis) relative to the thigh connection part 1A, as described above.

The present embodiment is described with reference to an example where, as shown in Fig. 2A, the thigh connection part 1A and the lower leg part 1B form part of a four-section link. The present embodiment, however, is not limited to such. As another example, the thigh connection part 1A and the lower leg part 1B may not form part of a four-section link. For example, as in the prosthetic knee joint described in the '339 Publication in Fig. 1, the lower leg part 1B may be coupled to the thigh connection part 1A such that the lower leg part 1B is rotatable around a real axis of rotation.

As shown in Figs. 1, 2A and 2B, the present embodiment is described with reference to an example case where the actuator of the prosthetic knee joint 1 is the cylinder 1C, which is a linearly movable actuator. The present embodiment, however, is not limited to such. As another example, the actuator of the prosthetic knee joint 1 may be an actuator of a different type, in place of a linearly movable actuator (the cylinder 1C), or the prosthetic knee joint 1 may include the linearly movable actuator and an additional actuator of a different type. As shown in Figs. 1, 2A and 2B, when the actuator is the cylinder 1C, which is a linearly movable actuator, the cylinder 1C can be, for example, a hydraulic or pneumatic type, or may be configured to use powders or magnetic fluids as the working fluid.

When the actuator of the prosthetic knee joint 1 includes the cylinder 1C, which is a linearly movable actuator, and an additional actuator of a different type, the additional actuator (for example, a rotary damper) may be, for example, a hydraulic or pneumatic type or configured to use powders as the working fluid. Here, working fluids of any types can be used. While the cylinder 1C is used as the linearly movable actuator in the present embodiment, a linear actuator (an actuator that uses a motor to achieve linear reciprocating motion) may be instead used, or an actuator that uses a rack-and-pinion structure to achieve linear reciprocating motion may be used.

The actuator may not only passively restrict or assist the rotational movement of the lower leg part 1B relative to the thigh connection part 1A, but may also actively rotate the lower leg part 1B around the axis of the knee relative to the thigh connection part 1A. In order to actively rotate the lower leg part 1B around the axis of the knee relative to the thigh connection part 1A, a high reduction ratio motor or the like may be used. When a high reduction ratio motor or the like is employed, the motor may be used to actively rotate the lower leg part 1B around the axis of the knee relative to the thigh connection part 1A, or, alternatively, to restrict the rotational movement of the lower leg part 1B relative to the thigh connection part 1A. Here, the speed reducer may be replaced with a pulley or the like.

In order to restrict the rotational movement of the lower leg part 1B relative to the thigh connection part 1A, a brake may be used, for example. The brake may be, for example, configured to sandwich the rotating shaft (i.e., preventing the rotating shaft from rotating), or to sandwich a piston rod of a cylinder (i.e., preventing the piston rod from reciprocating).

A clutch may be used to switch the resistance against the rotation of the lower leg part 1B against the thigh connection part 1A between a high rotation resistance state and a low rotation resistance state. For example, when the clutch is disengaged, the prosthetic knee joint 1 may enter the swing phase. On the other hand, when the clutch couples together the stationary part or slow rotating part and the lower leg part 1B, the prosthetic knee joint 1 may enter the stance phase. In order to switch the resistance against the rotation of the lower leg part 1B against the thigh connection part 1A between the high rotation resistance state and the low rotation resistance state, an electromagnetic force produced by, for example, an electromagnetic solenoid, may be used. In place of the cylinder 1C, which is a linearly movable actuator, an actuator with a McKibben-type artificial muscle such as a muscle suit, or an artificial muscle using magnetic force can be used, for example.

As shown in Figs. 1, 2A and 2B, the cylinder 1C is coupled to the thigh connection part 1A and the lower leg part 1B. The cylinder 1C restricts or assists the movement of the thigh connection part 1A (in detail, the rotational movement of the thigh connection part 1A relative to the lower leg part 1B). The cylinder 1C specifically includes a cylinder tube 1C1, a piston 1C2, and a piston rod 1C3.

Inside the cylinder tube 1C1, a first chamber 1C11 and a second chamber 1C12 are defined. The first chamber 1C11 is positioned on the side including the piston rod 1C3 with respect to the piston 1C2 (the upper side in Fig. 2B), and the second chamber 1C12 is positioned opposite the piston rod 1C3 with respect to the piston 1C2 (the lower side in Fig. 2B). The cylinder tube 1C1 has a coupling part 1C13 coupled to the lower leg part 1B. The piston rod 1C3 has a coupling part 1C31 coupled to the thigh connection part 1A.

The detector unit 1D directly or indirectly obtains information about how the thigh connection part 1A and the lower leg part 1B are relatively positioned. The detector unit 1D obtains information about how the thigh connection part 1A and the lower leg part 1B are relatively positioned by having a load sensor, a six-axis inertial sensor and a knee angle sensor disclosed in, for example, the '339 Publication in Fig. 2.

The present embodiment is, however, not limited to such. For example, the detector unit 1D may obtain information about how the thigh connection part 1A and the lower leg part 1B are relatively positioned by, for example, measuring the distance in a manner done by a position detection unit disclosed in Japanese Patent Application Publication No. 2019-180887 ("the '887 Publication") in Fig. 2. Additionally, the detector unit 1D may obtain information about how the thigh connection part 1A and the lower leg part 1B are relatively positioned by, for example, measuring the inclination angle in a manner done by the position detection unit disclosed in the '887 Publication in Fig. 6.

As shown in Figs. 1, 2A and 2B, the prosthetic knee joint 1 includes a control unit 1E, an estimating unit 1F, a stretching-side valve 1G, a stretching-side check valve 1G1, a bending-side valve 1H, a bending-side check valve 1H1, a processing unit 1I, a battery 1J, and a battery status acquiring unit 1K.

The control unit 1E controls the driving of the cylinder 1C based on the result detected by the detector unit 1D. The estimating unit 1F estimates the state of the user's movement based on the result detected by the detector unit 1D. The stretching-side valve 1G restricts extension of the cylinder 1C, thereby restricting stretching of the prosthetic knee joint 1. The opening of the stretching-side valve 1G is controlled by the control unit 1E.

As shown in Fig. 2B, as the opening of the stretching-side valve 1G decreases, this makes it difficult for the fluid in the first chamber 1C11 in the cylinder tube 1C1 to flow into the second chamber 1C12 through the stretching-side valve 1G. In other words, when the opening of the stretching-side valve 1G decreases, this prevents the cylinder 1C from extending, thereby preventing the prosthetic knee joint 1 from being stretched. In other words, when it is required to restrict the stretching of the prosthetic knee joint 1, the control unit 1E reduces the opening of the stretching-side valve 1G. When the stretching-side valve 1G is opened, the stretching-side check valve 1G1 prevents the fluid in the second chamber 1C12 from flowing back into the first chamber 1C11 through the stretching-side valve 1G.

The bending-side valve 1H restricts contraction of the cylinder 1C, thereby restricting bending of the prosthetic knee joint 1. The opening of the bending-side valve 1H is controlled by the control unit 1E. As shown in Fig. 2B, as the opening of the bending-side valve 1H decreases, this makes it difficult for the fluid in the second chamber 1C12 in the cylinder tube 1C1 to flow into the first chamber 1C11 through the bending-side valve 1H. In other words, when the opening of the bending-side valve 1H decreases, this prevents the cylinder 1C from contracting thereby preventing the prosthetic knee joint 1 from being bent. In other words, when it is required to restrict the bending of the prosthetic knee joint 1, the control unit 1E reduces the opening of the bending-side valve 1H. When the bending-side valve 1H is opened, the bending-side check valve 1H1 prevents the fluid in the first chamber 1C11 from flowing back into the second chamber 1C12 through the bending-side valve 1H.

The processing unit 1I performs various types of arithmetic and logical operations based on the results detected by the detector unit 1D. The battery 1J feeds power to the constituents making up the prosthetic knee joint 1 (in detail, the parts that consume power when operated), for example, the detector unit 1D, the control unit 1E, and the estimating unit 1F. The battery status acquiring unit 1K acquires the status of the battery 1J (e.g., the charge rate (SOC) (remaining power), the capacity, the open circuit voltage, the open circuit potential, etc.).

The estimating unit 1F estimates the state of the user's movement, more specifically, estimates whether the user is walking or not, based on the results detected by the detector unit 1D. When the estimating unit 1F estimates that the user is not walking, the control unit 1E places the prosthetic knee joint 1 in, for example, a maximum bending mode, a bicycle free mode, a seating free mode, a safety lock mode, a standing still mode, or the like.

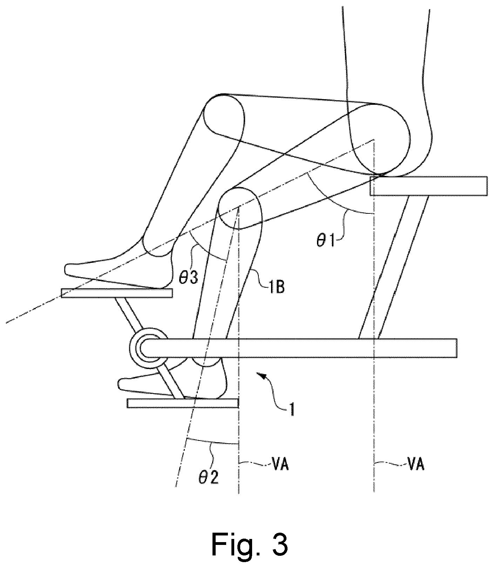

When the knee angle calculated by the processing unit 1I based on the results detected by the detector unit 1D is equal to or greater than a predetermined threshold value (specifically, 140 degrees), the control unit 1E places the prosthetic knee joint 1 in the maximum bending mode. The estimating unit 1F then estimates that the user is not walking. For example, the result "knee angle = 0 degrees" corresponds to the state shown in Fig. 2A. To be specific, the result "knee angle = 0 degrees" corresponds to the state where the prosthetic knee joint 1 is fully stretched (the user's thigh and the lower leg part 1B of the prosthetic knee joint 1 form a straight line, and the lower leg part 1B is located opposite the user's thigh with respect to the axis of the knee). For example, the result "knee angle = 160 degrees" corresponds to a state where the prosthetic knee joint 1 is bent. More specifically, the result "knee angle = 160 degrees" corresponds to a state where the user's thigh and the lower leg part 1B of the prosthetic knee joint 1 are bent, and the lower leg part 1B and the user's thigh are on the same side with respect to the axis of the knee (as shown in the `887 Publication in Fig. 3(d)).

When the user is not walking, it is not required to control the driving of the cylinder 1C as frequently (i.e., with as a short control cycle) as when the user is walking. Considering this, in the present embodiment, when the prosthetic knee joint 1 is placed in the maximum bending mode, the control unit 1E fully opens the stretching-side valve 1G so that the stretching of the prosthetic knee joint 1 not restricted, and fully opens the bending-side valve 1H so that the bending of the prosthetic knee joint 1 is not restricted. Furthermore, the control unit 1E sets a longer control cycle for the driving of the cylinder 1C than before the knee angle becomes 140 degrees or more. In other words, in the present embodiment, when the knee angle is 140 degrees or more and the estimating unit 1F estimates that the user is not walking, the control unit 1E sets a longer control cycle for the driving of the cylinder 1C than when the user is walking. This can improve the energy efficiency of the prosthetic knee joint 1 compared with a case where the control cycle for the driving of the cylinder 1C when the knee angle is 140 degrees or more is equal to the control cycle for the driving of the cylinder 1C before the knee angle reaches 140 degrees or more.

After the prosthetic knee joint 1 is placed in the maximum bending mode, the control unit 1E sets a shorter control cycle for the driving of the cylinder 1C than when the prosthetic knee joint 1 is placed in the maximum bending mode (for example, restores the control cycle employed for the driving of the cylinder 1C when the user is walking) when the knee angle falls below a predetermined threshold value (specifically, 135 degrees). As the control cycle for the driving of the cylinder 1C is changed, the detections may be done differently by the knee angle sensor, 6-axis inertial sensor, and load sensor, and the operations may be also performed differently by the processing unit 1I may be changed.

When all of the following conditions are satisfied, the control unit 1E places the prosthetic knee joint 1 in the bicycle free mode and the estimating unit 1F estimates that the user is not walking. The bicycle free mode is determined under the assumption that the user is riding a bicycle.
- If the minimum value of the thigh angle, which is calculated by the processing unit 1I based on the results detected by the detector unit 1D, becomes equal to or greater than a predetermined threshold value (specifically, 40 degrees).
- If the thigh angular velocity becomes equal to or greater than a predetermined threshold value (specifically, 30 degrees per second, hereinafter referred to as dps).
- If the knee angle becomes equal to or greater than a predetermined threshold value (specifically, 40 degrees).
- If the knee angular velocity becomes equal to or greater than a predetermined threshold value (40 dps).
- If the thigh angle is greater than the minimum value of the thigh angle by a predetermined threshold value (specifically, 10 degrees) or more, and the thigh angle becomes equal to or less than a predetermined threshold value (specifically, 65 degrees).

Stated differently, the estimating unit 1F estimates whether the state of the user's movement is the non-walking state or not, based on the minimum value of the thigh angle, thigh angular velocity, knee angle, knee angular velocity, and thigh angle, which are calculated by the processing unit 1I based on the results detected by the detector unit 1D. The minimum value of the thigh angle represents the value of the thigh angle observed when the thigh angle stops decreasing and start increasing. The minimum value of the thigh angle is reset to the above-described threshold value (40 degrees) when the thigh angle exceeds a predetermined threshold value (specifically, 70 degrees) or when the thigh angle decreases. When the thigh angle becomes smaller than the minimum value of the thigh angle, the minimum value of the thigh angle is updated.

Fig. 3 is used to illustrate, as an example, a case where the prosthetic knee joint 1 is placed in the bicycle free mode. In Fig. 3, the thigh angle θ1 represents the angle of inclination of the user's thigh relative to the vertical axis VA. In the present embodiment, the tilt angle θ2 of the lower leg part 1B relative to the vertical axis VA is detected by using the above-mentioned 6-axis inertial sensor as the detector unit 1D. When the above-mentioned knee angle sensor is used as the detector unit 1D, the knee angle θ3 is detected. Furthermore, the knee angular velocity is calculated by the processing unit 1I based on the results detected by the detector unit 1D. The thigh angle θ1 (= θ2 + θ3) and the thigh angular velocity are calculated by the processing unit 1I based on the tilt angle θ2 of the lower leg part 1B relative to the vertical axis VA and the knee angle θ3.

In the present embodiment, when the prosthetic knee joint 1 is placed in the bicycle free mode, the control unit 1E fully opens the stretching-side valve 1G so that the stretching of the prosthetic knee joint 1 is not restricted and fully opens the bending-side valve 1H so that the bending of the prosthetic knee joint 1 is not restricted. Furthermore, the control unit 1E sets a longer control cycle for the driving of the cylinder 1C than before the prosthetic knee joint 1 is placed in the bicycle free mode. In other words, in the present embodiment, when the prosthetic knee joint 1 is placed in the bicycle free mode, the estimating unit 1F estimates that the user is not walking, and the control unit 1E sets a longer control cycle for the driving of the cylinder 1C than when the user is walking. This can improve the energy efficiency of the prosthetic knee joint 1 compared with a case where the control cycle for the driving of the cylinder 1C when the prosthetic knee joint 1 is placed in the bicycle free mode is equal to the control cycle for the driving of the cylinder 1C when the user is walking.

After the prosthetic knee joint 1 is placed in the bicycle free mode, the control unit 1E sets a shorter control cycle for the driving of the cylinder 1C than when the prosthetic knee joint 1 is placed in the bicycle free mode (for example, restores the control cycle employed for the driving of the cylinder 1C when the user is walking) upon satisfaction of any of the following conditions.
- If the knee angle falls below a predetermined threshold value (specifically, 30 degrees), which is smaller than the above-mentioned threshold value (40 degrees) (i.e., if the knee is stretched)
- If the thigh angle falls below a predetermined threshold value (specifically, 10 degrees), which is smaller than the above-mentioned threshold value (65 degrees) (i.e., if the user's thigh is almost vertical and the user may possibly be walking)

If any of the following conditions are further satisfied, the control unit 1E places the prosthetic knee joint 1 in the seating free mode and the estimating unit 1F estimates that the user is not walking. The seating free mode is determined under the assumption that the user is seated on a chair.
- If the pitch angle of the lower leg part 1B, which is calculated by the processing unit 1I based on the results detected by the detector unit 1D, remains equal to or greater than a predetermined threshold value (specifically, 80 degrees) for a duration of a predetermined threshold value (specifically, 0.5 seconds) or more.
- If the thigh angle becomes equal to or greater than a predetermined threshold value (specifically, 70 degrees) and the knee angle becomes equal to or greater than a predetermined threshold value (specifically, 10 degrees).

In the present embodiment, when the prosthetic knee joint 1 is placed in the seating free mode, the control unit 1E fully opens the stretching-side valve 1G so that the stretching of the prosthetic knee joint 1 is not restricted, and fully opens the bending-side valve 1H so that the bending of the prosthetic knee joint 1 is not restricted. Furthermore, the control unit 1E sets a longer control cycle for the driving of the cylinder 1C than before the prosthetic knee joint 1 is placed in the seating free mode. In other words, in the present embodiment, when the prosthetic knee joint 1 is placed in the seating free mode, the estimating unit 1F estimates that the user is not walking and the control unit 1E sets a longer control cycle for the driving of the cylinder 1C than before the prosthetic knee joint 1 is placed in the seating free mode (specifically, than when the user is walking). This can improve the energy efficiency of the prosthetic knee joint 1 than when the control cycle for the driving of the cylinder 1C when the prosthetic knee joint 1 is placed in the seating free mode is equal to the control cycle for the driving of the cylinder 1C when the user is walking.

After the prosthetic knee joint 1 is placed in the seating free mode, the control unit 1E sets a shorter control cycle for the driving of the cylinder 1C than when the prosthetic knee joint 1 is placed in the seating free mode (for example, restores the control cycle employed for the driving of the cylinder 1C when the user is walking) upon satisfaction of any of the following conditions.
- If the pitch angle of the lower leg part 1B does not satisfy the condition that it remains equal to or greater than the above-mentioned threshold value (80 degrees) for a duration of the above-mentioned threshold value (0.5 seconds) or more, and the thigh angle is equal to or less than a predetermined threshold value (specifically, 65 degrees), which is smaller than the above-mentioned threshold value (70 degrees).
- If the knee angle is equal to or less than a predetermined threshold value (specifically, 7 degrees), which is smaller than the above-mentioned threshold value (10 degrees) (i.e., if the user may possibly be walking).

Furthermore, if any of the following conditions are satisfied, the control unit 1E places the prosthetic knee joint 1 in the standing still free mode and the estimating unit 1F estimates that the user is not walking. The standing still free mode is determined under the assumption that the user is standing and still (stationary while standing).
- If the knee angle calculated by the processing unit 1I based on the results detected by the detector unit 1D is less than a predetermined threshold value (specifically, 7 degrees) (i.e., the knee angle is small).
- If the forward tilt angle of the lower leg part 1B relative to the vertical axis is less than a predetermined threshold value (specifically, 3 degrees) or if the lower leg part 1B is tilted backward (that is, if the lower leg part 1B is tilted backward relative to the forward tilt angle of 3 degrees).
- If the angular velocity of the lower leg part 1B on the forward tilt side relative to the vertical axis is equal to or less than a predetermined threshold value (specifically, 100 dps), or if the backward tilt angle of the lower leg part 1B is increasing (that is, if the forward tilt angle of the lower leg part 1B is not increasing at a high rate).
- If the angular velocity of the lower leg part 1B making an angle in the front-rear direction relative to the vertical axis is equal to or less than a predetermined threshold value (specifically, 200 dps) (i.e., if neither the forward nor the backward tilt angle of the lower leg part 1B is increasing significantly).

In the present embodiment, when the prosthetic knee joint 1 is placed in the standing still mode, the control unit 1E places the stretching-side valve 1G in a pre-adjusted state between the fully open state and the fully closed state and places the bending-side valve 1H in a yielding state between the fully open state and the fully closed state. Furthermore, the control unit 1E sets a longer control cycle for the driving of the cylinder 1C than before the prosthetic knee joint 1 is placed in the standing still mode. In other words, in the present embodiment, when the prosthetic knee joint 1 is placed in the standing still mode, the estimating unit 1F estimates that the user is not walking and the control unit 1E sets a longer control cycle for the driving of the cylinder 1C than before the prosthetic knee joint 1 is placed in the standing still mode (specifically, than when the user is walking). This can improve the energy efficiency of the prosthetic knee joint 1 compared with a case where the control cycle for the driving of the cylinder 1C when the prosthetic knee joint 1 is placed in the standing still mode is equal to the control cycle for the driving of the cylinder 1C when the user is walking.

After the prosthetic knee joint 1 is placed in the standing still mode, the control unit 1E sets a shorter control cycle for the driving of the cylinder 1C than when the prosthetic knee joint 1 is placed in the standing still mode (for example, restores the control cycle employed for the driving of the cylinder 1C when the user is walking) upon satisfaction of all of the following conditions.
- If the knee angle becomes equal to or greater than the above-mentioned threshold value (7 degrees).
- If the forward tilt angle of the lower leg part 1B relative to the vertical axis becomes equal to or greater than a predetermined threshold value (specifically, 5 degrees), which is larger than the above-mentioned threshold value (3 degrees).
- If the angular velocity of the lower leg part 1B on the forward tilt side relative to the vertical axis becomes equal to or greater than a predetermined threshold value (specifically, 50 dps), or if the absolute value of the angular velocity of the lower leg part 1B in the front-rear direction relative to the vertical axis becomes equal to or greater than a predetermined threshold value (specifically, 300 dps) (that is, if the user may possibly be walking).

When all of the following conditions are further satisfied, the control unit 1E places the prosthetic knee joint 1 in the safety lock mode and the estimating unit 1F estimates that the user is not walking. The safety lock mode is determined under the assumption that the user is half sitting.
- If the absolute value of the tilt angle of the lower leg part 1B in the front-rear direction relative to the vertical axis calculated by the processing unit 1I based on the results detected by the detector unit 1D becomes equal to or less than a predetermined threshold value (specifically, 30 degrees).
- If the absolute value of the angular velocity of the lower leg part 1B making an angle in the front-rear direction relative to the vertical axis becomes equal to or less than a predetermined threshold value (specifically, 200 dps)
- If the knee angle falls within a range from a predetermined threshold value (specifically, 7 degrees) to a predetermined threshold value (specifically, 60 degrees).
- If the absolute value of the knee angular velocity becomes equal to or less than a predetermined threshold value (specifically, 12 dps)

In the present embodiment, when the prosthetic knee joint 1 is placed in the safety lock mode, the control unit 1E fully opens the stretching-side valve 1G so that the stretching of the prosthetic knee joint 1 is not restricted, and fully closes the bending-side valve 1H in a fully closed state so that the bending of the prosthetic knee joint 1 is restricted. Furthermore, the control unit 1E sets a longer control cycle for the driving of the cylinder 1C than before the prosthetic knee joint 1 is placed in the safety lock mode. In other words, in the present embodiment, when the prosthetic knee joint 1 is placed in the safety lock mode, the estimating unit 1F estimates that the user is not walking and the control unit 1E sets a longer control cycle for the driving of the cylinder 1C than before the prosthetic knee joint 1 is placed in the safety lock mode (specifically, than when the user is walking). This can improve the energy efficiency of the prosthetic knee joint 1 than when the control cycle for the driving of the cylinder 1C when the prosthetic knee joint 1 is placed in the safety lock mode is equal to the control cycle for the driving of the cylinder 1C when the user is walking.

After the prosthetic knee joint 1 is placed in the safety lock mode, the control unit 1E sets a shorter control cycle for the driving of the cylinder 1C than when the prosthetic knee joint 1 is placed in the safety lock mode (for example, restores the control cycle employed for the driving of the cylinder 1C when the user is walking) upon satisfaction of any of the following conditions.
- If the absolute value of the tilt angle of the lower leg part 1B in the front-rear direction relative to the vertical axis becomes greater than the above-mentioned threshold value (30 degrees) (i.e., if the user may possibly be walking.)
- If the absolute value of the angular velocity of the lower leg part 1B making an angle in the front-rear direction relative to the vertical axis becomes greater than the above-mentioned threshold value (200 dps) (i.e., if the user may possibly be walking).
- If the knee angle becomes less than the above-mentioned threshold value (7 degrees) (i.e., if the user may possibly be walking).
- If the knee angle becomes greater than the above-mentioned threshold value (60 degrees) (i.e., if the user may possibly be walking).
- If the absolute value of the angular velocity of the knee becomes greater than the above-mentioned threshold (12 dps) (i.e., if the user may possibly be walking).

To make it more difficult for the prosthetic knee joint 1 to exit the safety lock mode, the prosthetic knee joint 1 may remain in the safety lock mode if the angular velocity of the knee falls within the range between 40 dps on the prosthetic knee joint 1 stretching side and 20 dps on the prosthetic knee joint 1 bending side.

As described above, according to the prosthetic knee joint 1 of the present embodiment, when the estimating unit 1F estimates that the user is not walking, the control unit 1E sets a longer control cycle for the driving of the cylinder 1C than when the estimating unit 1F estimates that the user is walking. This can improve the energy efficiency of the prosthetic knee joint 1, when compared with the case where the control cycle for the driving of the cylinder 1C when the user is not waling is equal to the control cycle for the driving of the cylinder 1C when the user is walking. In other words, when the knee angle calculated based on the results detected by the detector unit 1D falls within a predetermined range, the control unit 1E sets a longer control cycle for the driving of the cylinder 1C than when the knee angle is outside the predetermined range.

More specifically, according to the prosthetic knee joint 1 of the present embodiment, when the estimating unit 1F estimates that the user is not walking, the control unit 1E performs a first control such that the processing unit 1I performs the operations less frequently than when the estimating unit 1F estimates that the user is walking. Additionally, when the estimating unit 1F estimates that the user is not walking, the control unit 1E performs a second control such that part of the control performed when the estimating unit 1F estimates that the user is walking is prohibited. Furthermore, when the estimating unit 1F estimates that the user is not walking, the control unit 1E performs a third control such that the control unit 1E obtains the results detected by the detector unit 1D with a longer cycle than when the estimating unit 1F estimates that the user is walking.

The present embodiment, however, is not limited to such. As another example, when the estimating unit 1F estimates that the user is not walking, the control unit 1E may not perform all of the first, second and third controls. In this case, for example, the control unit 1E may perform at least one of the first control, the second control, or the third control.

Furthermore, according to the prosthetic knee joint 1 of the present embodiment, the control unit 1E sets a longer control cycle for the driving of the cylinder 1C not only based on the estimation made by the estimating unit 1F, but also based on the status of the battery 1J acquired by the battery status acquiring unit 1K. For example, when the estimating unit 1F estimates that the user is not walking and the battery 1J has low remaining power, the control unit 1E sets a longer control cycle for the driving of the cylinder 1C than when the estimating unit 1F estimates that the user is not walking and the battery 1J has high remaining power. This can contribute to further reduce the power consumption when the remaining power of the battery 1J is low, so that the battery 1J can be charged less frequently.

### <Second Embodiment>

The following describes a prosthetic knee joint relating to a second embodiment of the present disclosure. The prosthetic knee joint 1 of the second embodiment is configured in the same manner as the prosthetic knee joint 1 of the above-described first embodiment except for the following differences. Accordingly, the prosthetic knee joint 1 of the second embodiment can produce the same effects as the prosthetic knee joint 1 of the above-described first embodiment except for the following differences.

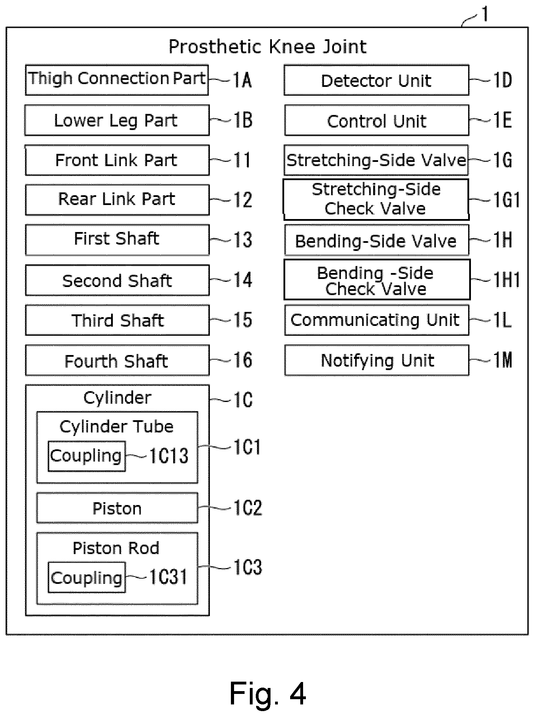

Fig. 4 shows an example configuration of the prosthetic knee joint 1 according to the second embodiment of the invention. As shown in Fig. 4, the prosthetic knee joint 1 of the second embodiment includes the thigh connection part 1A, the lower leg part 1B, the front link part 11, the rear link part 12, the first shaft 13, the second shaft 14, the third shaft 15, the fourth shaft 16, the cylinder 1C acting as an actuator, and the detector unit 1D, similarly to the prosthetic knee joint 1 of the first embodiment. The thigh connection part 1A is connectable to a socket configured to receive the thigh of the user (the wearer of the prosthetic knee joint 1). The lower leg part 1B is coupled to the thigh connection part 1A such that the lower leg part 1B is rotatable around the axis of the knee.

In the present embodiment, as is the case shown in Fig. 2A, the thigh connection part 1A and the lower leg part 1B form part of a four-section link. The present embodiment, however, is not limited to such. As another example, the thigh connection part 1A and the lower leg part 1B may not form part of a four-section link. The lower leg part 1B may be coupled to the thigh connection part 1A such that the lower leg part 1B is rotatable around a real axis of rotation. In the present embodiment, as in the first embodiment, the actuator restricts or assists the movement of the thigh connection part 1A.

In the present embodiment, the prosthetic knee joint 1 includes, as the actuator, the cylinder 1C, which is a linearly movable actuator. The present embodiment, however, is not limited to such. As another example, the actuator of the prosthetic knee joint 1 may be an actuator of a different type, instead of a linearly movable actuator (the cylinder 1C), or the prosthetic knee joint 1 may include the linearly movable actuator and an additional actuator of a different type.

When the actuator is the cylinder 1C, which is a linearly movable actuator, in the present embodiment, the cylinder 1C can be, for example, a hydraulic or pneumatic type, or may be configured to use powders or magnetic fluids as the working fluid. All of the actuators listed, as an example, applicable to the prosthetic knee joint 1 of the first embodiment are also applicable to the prosthetic knee joint 1 of the second embodiment.

In the present embodiment, the detector unit 1D detects the state of the user's movement. The detector unit 1D detects the state of the user's movement by having, for example, a knee angle sensor and a load sensor. The present embodiment, however, is not limited to such. As another example, the detector unit 1D may detect the state of the user's movement by having a knee angle sensor, a 6-axis inertial sensor, etc.

As shown in Fig. 4, the prosthetic knee joint 1 includes the control unit 1E, the stretching-side valve 1G, the stretching-side check valve 1G1, the bending-side valve 1H, the bending-side check valve 1H1, a communicating unit 1L and a notifying unit 1M. The control unit 1E controls the driving of the cylinder 1C based on the results detected by the detector unit 1D. The communicating unit 1L communicates with an external device (e.g., a terminal device carried by the user). The notifying unit 1M issues a notification when the communicating unit 1L is enabled to communicate with the external device.

For example, the notifying unit 1M issues a notification to the user using a motor for generating vibration, a buzzer, and a light-emitting device. This allows the user to know that the communicating unit 1L is enabled to communicate with the external device. The present embodiment, however, is not limited to such. As another example, the notification by the notifying unit 1M may be performed by the communicating unit 1L sending to the external device the notification indicating that the communicating unit 1L is enabled to communicate with the external device.

In the present embodiment, the prosthetic knee joint 1 is movable as indicated by the user's manipulation entered through the manipulation screen of the external device (for example, the user changes the mode of the prosthetic knee joint 1), and capable of outputting the state of the prosthetic knee joint 1 (for example, the status of the battery) to the external device. To implement these capabilities, wireless communication is used between the prosthetic knee joint 1 and the external device.

Here, when the user of the prosthetic knee joint 1 takes an airplane, the wireless communication function between the prosthetic knee joint 1 and the external device is turned off during the taking-off and landing of the airplane. After the airplane takes off and lands, the user may desire to turn on the wireless communication function between the prosthetic knee joint 1 and the external device. To cope with this issue, the prosthetic knee joint 1 according to the second embodiment has the following function so that the user can easily turn on the wireless communication function between the prosthetic knee joint 1 and the external device after the taking-off and landing of the airplane, for example. The communicating unit 1L is enabled to communicate with the external device (the wireless communication function is turned on) when the detector unit 1D detects that the user's movement is in a predetermined state.

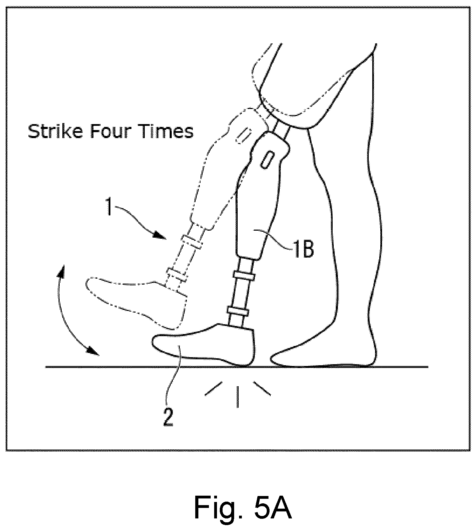

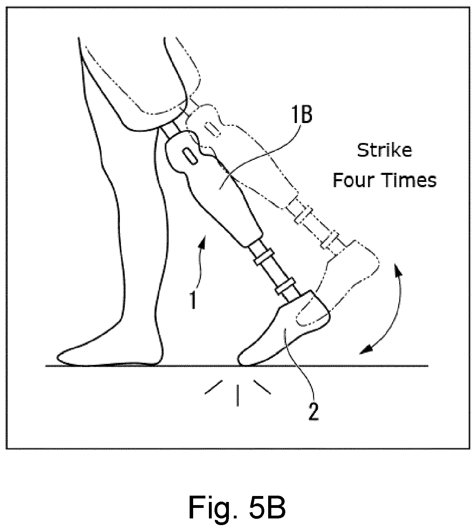

Figs. 5A and 5B show examples of the user's movement required to turn on the wireless communication function between the prosthetic knee joint 1 and the external device. More specifically, Fig. 5A shows a first example of the user's movement required to turn on the wireless communication function. Fig. 5B shows a second example of the user's movement required to turn on the wireless communication function.

According to the first example shown in Fig. 5A, the wireless communication function between the communicating unit 1L of the prosthetic knee joint 1 and the external device is turned on by the user of the prosthetic knee joint 1 striking the ground or floor surface with the heel part of the foot part 2 connected to the lower leg part 1B of the prosthetic knee joint 1 a predetermined number of times (e.g., four times). The detector unit 1D of the prosthetic knee joint 1 detects the state of the user's movement (specifically, the user striking the ground or floor surface with the heel part of the foot part 2 the predetermined number of times). Here, "the predetermined number of times" is set such that the user would normally never strike the ground or floor surface the predetermined number of times while walking.

The communicating unit 1L has, in advance, information indicating that the user's movement required to turn on the wireless communication function is the user striking the ground or floor surface with the heel part of the foot part 2 the predetermined number of times (information indicating the condition for turning on the wireless communication function). According to the first example shown in Fig. 5A, the communicating unit 1L is enabled to communicate with the external device (the wireless communication function is turned on) since the detector unit 1D detects that the user's movement is in the state required to turn on the wireless communication function. As a result, the user can manipulate the prosthetic knee joint 1 via the manipulation screen of the external device. In addition, the user can output the state of the prosthetic knee joint 1 to the external device.

According to the second example shown in Fig. 5B, the wireless communication function between the communicating unit 1L of the prosthetic knee joint 1 and the external device is turned on by the user of the prosthetic knee joint 1 striking the ground or floor surface with the toe part of the foot part 2 connected to the lower leg part 1B of the prosthetic knee joint 1 a predetermined number of times (e.g., four times). The detector unit 1D of the prosthetic knee joint 1 detects the state of the user's movement (specifically, the user striking the ground or floor surface with the toe part of the foot part 2 the predetermined number of times). The communicating unit 1L has, in advance, information indicating that the user's movement required to turn on the wireless communication function is the user striking the ground or floor surface with the toe part of the foot part 2 the predetermined number of times (information indicating the condition for turning on the wireless communication function).

According to the second example shown in Fig. 5B, the communicating unit 1L is enabled to communicate with the external device (the wireless communication function is turned on) since the detector unit 1D detects that the user's movement is in the state required to turn on the wireless communication function. As a result, the user can manipulate the prosthetic knee joint 1 via the operation screen of the external device. In addition, the user can output the state of the prosthetic knee joint 1 to the external device.

As described above, the user can select, on the manipulation screen of the external device, either striking the ground or floor surface with the heel part of the foot part 2 the predetermined number of times, or striking the ground or floor surface with the toe part of the foot part 2 the predetermined number of times, as the user's movement required to turn on the wireless communication function.

In the first and second examples shown in Figs. 5A and 5B, the detector unit 1D has a load sensor (i.e., detects the load applied to the prosthetic knee joint 1) to detect the state of the user's movement. The present embodiment, however, is not limited to such. As another example, the detector unit 1D may detect the state of the user's movement by having a 6-axis inertial sensor, etc. (i.e., by detecting the acceleration of the prosthetic knee joint 1).

According to another example, the communication between the communicating unit 1L and the external device may be enabled if the communication between the communicating unit 1L and the external device is temporarily interrupted and the communicating unit 1L is then restarted. In other words, the communicating unit 1L may be enabled to communicate with the external device (the wireless communication function is turned on) if the communicating unit 1L is restarted after the communication between the communicating unit 1L and the external device is temporarily cut off, for example, due to communication errors, during an airplane taking-off or landing and the like.

According to another example, when the user taps the side surface of the prosthetic knee joint 1 (i.e., when the detector unit 1D detects acceleration in the left-right direction), the communicating unit 1L may be enabled to communicate with the external device (the wireless communication function may be turned on). According to another example, the communicating unit 1L may be enabled to communicate with the external device (the wireless communication function may be turned on) when the detector unit 1D detects that, for example, the user unbuckles the seatbelt and stands up after an airplane takes off or lands. According to another example, the communicating unit 1L may be enabled to communicate with the external device (the wireless communication function may be turned on) when the detector unit 1D detects that, for example, the user unbuckles the seatbelt and starts walking after an airplane takes off or lands.

According to the foregoing embodiments disclosed herein, a plurality of functions are distributively provided. Some or all of the functions may be integrated. Any one of the functions may be partly or entirely segmented into a plurality of functions, which are distributively provided. Irrespective of whether or not the functions are integrated or distributed, they are acceptable as long as they are configured to solve the problems. The foregoing is the detailed description of the embodiments of the present invention with reference to the drawings. The specific configurations are not limited to the above embodiments and examples but include design modifications within the purport of the present invention. The embodiments and examples described above may be combined with each other.

### LIST OF REFERENCE NUMBERS

1 ···prosthetic knee joint, 1A···thigh connection part, 1B···lower leg part, 11···front link part, 12···rear link part, 13···first shaft, 14···second shaft, 15···third shaft, 16···fourth shaft, 1C···cylinder, 1C1 ···cylinder tube, 1C11···-first chamber, 1C12···second chamber, 1C13···coupling part, 1C2···piston, 1C3···piston rod, 1C31···-coupling part, 1D···detector unit, 1E···control unit, 1F···estimating unit, 1G···stretching-side valve, 1G1···stretching-side check valve, 1H···bending-side valve, 1H1···bending-side check valve, 1I···processing unit, 1J···battery, 1K···battery status obtaining unit, 1L···communicating unit, 1M···notifying unit, 2 ... foot part, Θ1···thigh angle, Θ2···tilt angle, Θ3··knee angle, VA···vertical axis

## Claims

1. A prosthetic knee joint (1) comprising:
a thigh connection part (1A) connectable to a socket configured to receive a thigh of a user;
a lower leg part (1B) coupled to the thigh connection part (1A) such that the lower leg part (1B) is rotatable around an axis of a knee;
an actuator (1C) coupled to the thigh connection part (1A) and the lower leg part (1B), the actuator (1C) being configured to restrict or assist movement of the thigh connection part (1A);
a detector unit (1D) for directly or indirectly obtaining information about how the thigh connection part (1A) and the lower leg part (1B) are relatively positioned;
a control unit (1E) for controlling driving of the actuator (1C) based on a result detected by the detector unit (1D); and
an estimating unit (1F) for estimating a state of the user's movement based on the result detected by the detector unit (1D),
**characterized in that**, when
the estimating unit (1F) estimates that the user is not walking, the control unit (1E) sets a longer control cycle for the driving of the actuator (1C) than when the estimating unit (1F) estimates that the user is walking.

2. The prosthetic knee joint (1) of claim 1, wherein the estimating unit (1F) estimates that the user is not walking when a knee angle calculated based on the result detected by the detector unit (1D) is equal to or greater than a first threshold value.

3. The prosthetic knee joint (1) of claim 1, wherein the estimating unit (1F) estimates that the user is not walking when a minimum value of a thigh angle, a thigh angular velocity, a knee angle, a knee angular velocity, and a thigh angle calculated based on the result detected by the detector unit (1D) satisfy a predetermined threshold value.

4. The prosthetic knee joint (1) of claim 1,
wherein a first condition is defined such that a pitch angle of the lower leg part (1B) calculated based on the result detected by the detector unit (1D) remains equal to or greater than a second threshold value or more for a duration of a third threshold value or more,
wherein a second condition is defined such that a thigh angle becomes equal to or greater than a fourth threshold value and a knee angle becomes equal to or greater than a fifth threshold value, and
wherein the estimating unit (1F) estimates that the user is not walking if the first or second condition is satisfied.

5. The prosthetic knee joint (1) of claim 1,
wherein a third condition is defined such that a knee angle calculated based on the result detected by the detector unit (1D) becomes less than a sixth threshold value,
wherein a fourth condition is defined such that a forward tilt angle of the lower leg part (1B) relative to a vertical axis is less than a seventh threshold value or the lower leg part (1B) is tilted backward,
wherein a fifth condition is defined such that an angular velocity of the lower leg part (1B) on a forward tilt side relative to the vertical axis is equal to or less than an eighth threshold value, or a backward tilt angle of the lower leg part (1B) is increasing,
wherein a sixth condition is defined such that an angular velocity of the lower leg part (1B) making an angle in a front-rear direction relative to the vertical axis becomes equal to or less than a ninth threshold value, and
wherein the estimating unit (1F) estimates that the user is not walking if any one of the third to sixth conditions is satisfied.

6. The prosthetic knee joint (1) of claim 1,
wherein a seventh condition is defined such that an absolute value of a tilt angle of the lower leg part (1B) in a front-rear direction relative to a vertical axis calculated based on the result detected by the detector unit (1D) becomes equal to or less than a tenth threshold value,
wherein an eighth condition is defined such that an absolute value of an angular velocity of the lower leg part (1B) making an angle in the front-rear direction relative to the vertical axis becomes equal to or less than an eleventh threshold,
wherein a ninth condition is defined such that a knee angle falls within a range from a twelfth threshold value to a thirteenth threshold value,
wherein a tenth condition is defined such that an absolute value of a knee angular velocity becomes equal to or less than a fourteenth threshold value, and
wherein the estimating unit (1F) estimates that the user is not walking if all of the seventh to tenth conditions are satisfied.

7. The prosthetic knee joint (1) of claim 1,
wherein a processing unit (1I) is configured to perform operations based on the result detected by the detector unit (1D), and a first process is defined as controlling the processing unit (1I) such that the processing unit (1I) performs the operations less frequently than when the estimating unit (1F) estimates that the user is walking,
wherein a second process is defined as interrupting a part of a control that is to be performed when the estimating unit (1F) estimates that the user is walking,
wherein a third process is defined as setting a longer cycle for obtaining the result detected by the detector unit (1D) than when the estimating unit (1F) estimates that the user is walking, and
wherein the control unit (1E) performs at least one of the first process, the second process or the third process when the estimating unit (1F) estimates that the user is not walking.

8. The prosthetic knee joint (1) of claim 1, further comprising a battery status acquiring unit (1K) for acquiring a status of a battery (1J) provided for feeding power to the detector unit (1D), the control unit (1E), and the estimating unit (1F),
wherein the control unit (1E) sets a longer control cycle for the driving of the actuator (1C) based on the estimation made by the estimating unit (1F) and the state of the battery (1J) acquired by the battery status acquiring unit (1K).

9. The prosthetic knee joint (1) of claim 1,
wherein, when a knee angle calculated based on the result detected by the detector unit (1D) is within a predetermined range, the control unit (1E) sets a longer control cycle for the driving of the actuator (1C) than when the knee angle is outside the predetermined range.

10. The prosthetic knee joint (1) of claim 9, further comprising:
a stretching-side valve (1G) for restricting stretching of the prosthetic knee joint (1) by restricting extension of the actuator (1C); and
a bending-side valve (1H) for restricting bending of the prosthetic knee joint (1) by restricting contraction of the actuator (1C),
wherein, when the knee angle reaches 140 degrees or greater, the control unit (1E) fully opens the stretching-side valve (1G) so that the stretching of the prosthetic knee joint (1) is not restricted, fully opens the bending-side valve (1H) so that the bending of the prosthetic knee joint (1) is not restricted, and sets a longer control cycle for the driving of the actuator (1C) than before the knee angle reaches 140 degrees or greater.

11. The prosthetic knee joint (1) of claim 9, further comprising:
a stretching-side valve (1G) for restricting stretching of the prosthetic knee joint (1) by restricting extension of the actuator (1C); and
a bending-side valve (1H) for restricting bending of the prosthetic knee joint (1) by restricting contraction of the actuator (1C),
wherein an eleventh condition is defined such that a minimum value of a thigh angle calculated based on the result detected by the detector unit (1D) becomes equal to or greater than a fifteenth threshold value,
wherein a twelfth condition is defined such that a thigh angular velocity becomes equal to or greater than a sixteenth threshold value,
wherein a thirteenth condition is defined such that the knee angle becomes equal to or greater than a seventeenth threshold value,
wherein a fourteenth condition is defined such that a knee angular velocity becomes equal to or greater than an eighteenth threshold value,
wherein a fifteenth condition is defined such that a thigh angle is greater than the minimum value of the thigh angle by a nineteenth threshold value or more and the thigh angle becomes equal to or less than a twentieth threshold value,
wherein the control unit (1E) places the prosthetic knee joint (1) in a bicycle free mode when all of the eleventh to fifteenth conditions are satisfied, and
wherein the control unit (1E) further sets a longer control cycle for the driving of the actuator (1C) than before the prosthetic knee joint (1) is placed in the bicycle free mode, fully opens the stretching-side valve (1G) so that the stretching of the prosthetic knee joint (1) is not restricted, and fully opens the bending-side valve (1H) so that the bending of the prosthetic knee joint (1) is not restricted.

12. The prosthetic knee joint (1) of claim 11,
wherein a sixteenth condition is defined such that the knee angle becomes less than a twenty-first threshold value that is less than the seventeenth threshold value,
wherein a seventeenth condition is defined such that the thigh angle becomes less than a twenty-second threshold value that is less than the twentieth threshold value, and
wherein, after the prosthetic knee joint (1) is placed in the bicycle free mode, the control unit (1E) sets a shorter control cycle for the driving of the actuator (1C) than when the prosthetic knee joint (1) is placed in the bicycle free mode upon satisfaction of the sixteenth or seventeenth condition.

13. The prosthetic knee joint (1) of claim 9, further comprising:
a stretching-side valve (1G) for restricting stretching of the prosthetic knee joint (1) by restricting extension of the actuator (1C); and
a bending-side valve (1H) for restricting bending of the prosthetic knee joint (1) by restricting contraction of the actuator (1C),
wherein an eighteenth condition is defined such that a pitch angle of the lower leg part (1B) remains equal to or greater than a twenty-third threshold value for a duration of a twenty-fourth threshold value or more,
wherein a nineteenth condition is defined such that a thigh angle becomes equal to or greater than a twenty-fifth threshold value and the knee angle becomes equal to or greater than a twenty-sixth threshold value,
wherein a twentieth condition is defined such that (i) the condition that the pitch angle of the lower leg part (1B) remains equal to or greater than the twenty-third threshold value for the duration of the twenty fourth threshold value or more is not satisfied and (ii) the thigh angle becomes equal to or less than a twenty-seventh threshold value that is smaller than the twenty-fifth threshold value,
wherein a twenty-first condition is defined such that the knee angle becomes equal to or less than a twenty-eighth threshold value that is less than the twenty-sixth threshold value,
wherein the control unit (1E) performs at least a first control selected from the first control and a second control,
wherein, when performing the first control, the control unit (1E) places the prosthetic knee joint (1) in a seating free mode when the eighteenth or nineteenth condition is satisfied, and sets a longer control cycle for the driving of the actuator (1C) than before the prosthetic knee joint (1) is placed in the seating free mode, fully opens the stretching-side valve (1G) so that the stretching of the prosthetic knee joint (1) is not restricted, and fully opens the bending-side valve (1H) so that the bending of the prosthetic knee joint (1) is not restricted, and
wherein, when performing the second control, the control unit (1E) sets, after the prosthetic knee joint (1) is placed in the seating free mode, a shorter control cycle for the driving of the actuator (1C) than when the prosthetic knee joint (1) is placed in the seating free mode upon satisfaction of the twentieth or twenty-first condition

14. The prosthetic knee joint (1) of claim 9, further comprising:
a stretching-side valve (1G) for restricting stretching of the prosthetic knee joint (1) by restricting extension of the actuator (1C); and
a bending-side valve (1H) for restricting bending of the prosthetic knee joint (1) by restricting contraction of the actuator (1C),
wherein a twenty-second condition is defined such that the knee angle becomes less than a twenty-ninth threshold value,
wherein a twenty-third condition is defined such that a forward tilt angle of the lower leg part (1B) relative to a vertical axis is less than a thirtieth threshold value or the lower leg part (1B) is tilted backward,
wherein a twenty-fourth condition is defined such that an angular velocity of the lower leg part (1B) on a forward tilt side relative to the vertical axis is equal to or less than a thirty-first threshold value, or a backward tilt angle of the lower leg part (1B) is increasing,
wherein a twenty-fifth condition is defined such that an angular velocity of the lower leg part (1B) making an angle in a front-rear direction relative to the vertical axis becomes equal to or less than a thirty-second threshold value,
wherein a twenty-sixth condition is defined such that the knee angle becomes equal to or greater than the twenty-ninth threshold value,
wherein a twenty-seventh condition is defined such that a forward tilt angle of the lower leg part (1B) relative to the vertical axis becomes equal to or greater than a thirty-third threshold value that is greater than the thirtieth threshold value,
wherein a twenty-eighth condition is defined such that an angular velocity of the lower leg part (1B) on a forward tilt side relative to the vertical axis becomes equal to or greater than a thirty-fourth threshold value, or an absolute value of an angular velocity of the lower leg part (1B) in the front-rear direction relative to the vertical axis become equal to or greater than a thirty-fifth threshold value,
wherein the control unit (1E) performs at least a third control selected from the third control and a fourth control,
wherein, when performing the third control, the control unit (1E) places the prosthetic knee joint (1) in a standing still mode when any one of the twenty-second to twenty-fifth conditions is satisfied, and sets a longer control cycle for the driving of the actuator (1C) than before the prosthetic knee joint (1) is placed in the standing still mode and places the stretching-side valve (1G) in a pre-adjusted state between a fully open state and a fully closed state and places the bending-side valve (1H) in a yielding state between the fully open state and the fully closed state, and
wherein, when performing the fourth control, the control unit (1E) sets, after the prosthetic knee joint (1) is placed in the standing still mode, a shorter control cycle for the driving of the actuator (1C) than when the prosthetic knee joint (1) is placed in the standing still mode upon satisfaction of all of the twenty-sixth to twenty-eighth conditions.

15. The prosthetic knee joint (1) of claim 9, further comprising:
a stretching-side valve (1G) for restricting stretching of the prosthetic knee joint (1) by restricting extension of the actuator (1C); and
a bending-side valve (1H) for restricting bending of the prosthetic knee joint (1) by restricting contraction of the actuator (1C),
wherein a twenty-ninth condition is defined such that an absolute value of a tilt angle of the lower leg part (1B) in a front-rear direction relative to a vertical axis is equal to or less than a thirty-sixth threshold,
wherein a thirtieth condition is defined such that an absolute value of an angular velocity of the lower leg part (1B) making an angle in the front-rear direction relative to the vertical axis becomes equal to or less than a thirty-seventh threshold value,
wherein a thirty-first condition is defined such that the knee angle falls within a range from a thirty-eighth threshold value to a thirty-ninth threshold value,
wherein a thirty-second condition is defined such that an absolute value of a knee angular velocity becomes equal to or less than a fortieth threshold value,
wherein a thirty-third condition is defined such that the absolute value of the tilt angle of the lower leg part (1B) in the front-rear direction relative to the vertical axis becomes greater than the thirty-sixth threshold value,
wherein a thirty-fourth condition is defined such that an absolute value of an angular velocity of the lower leg part (1B) making an angle in the front-rear direction relative to the vertical axis becomes greater than the thirty-seventh threshold value,
wherein a thirty-fifth condition is defined such that the knee angle becomes less than the thirty-eighth threshold value,
wherein a thirty-sixth condition is defined such that the knee angle becomes greater than the thirty-ninth threshold value or an absolute value of the knee angular velocity becomes greater than the fortieth threshold value,
wherein the control unit (1E) performs at least a fifth control selected from the fifth control and a sixth control,
wherein, when performing the fifth control, the control unit (1E) places the prosthetic knee joint (1) in a safety lock mode when all of the twenty-ninth to thirty-second conditions are satisfied, and sets a longer control cycle for the driving of the actuator (1C) than before the prosthetic knee joint (1) is placed in the safety lock mode, fully opens the stretching-side valve (1G) so that the stretching of the prosthetic knee joint (1) is not restricted, and fully closes the bending-side valve (1H) so that the bending of the prosthetic knee joint (1) is restricted, and
wherein, when performing the sixth control, the control unit (1E) sets, after the prosthetic knee joint (1) is placed in the safety lock mode, a shorter control cycle for the driving of the actuator (1C) than when the prosthetic knee joint (1) is placed in the safety lock mode upon satisfaction of any one of the thirty-third to thirty-sixth conditions.

## Patentansprüche

1. Kniegelenkprothese (1), die umfasst:
einen Oberschenkel-Verbindungsteil (1A), der mit einer Aufnahme verbunden werden kann, die zum Aufnehmen eines Oberschenkels eines Benutzers ausgeführt ist;
einen Unterschenkelteil (1B), der mit dem Oberschenkel-Verbindungsteil (1A) so gekoppelt ist, dass der Unterschenkelteil (1B) um eine Achse eines Knies herum gedreht werden kann;
ein Stellglied (1C), das mit dem Oberschenkel-Verbindungsteil (1A) und dem Unterschenkelteil (1B) gekoppelt ist, wobei das Stellglied (1C) so ausgeführt ist, dass es Bewegung des Oberschenkel-Verbindungsteils (1A) einschränkt oder unterstützt;
eine Detektor-Einheit (1D) zum direkten oder indirekten Ermitteln von Informationen darüber, wie der Oberschenkel-Verbindungsteil (1A) und der Unterschenkelteil (1B) relativ zueinander positioniert sind;
eine Steuerungs-Einheit (1E) zum Steuern von Antrieb des Stellgliedes (1C) auf Basis eines von der Detektor-Einheit (1D) erfassten Ergebnisses; und
eine Schätz-Einheit (1F) zum Schätzen eines Zustandes der Bewegung des Benutzers auf Basis des von der Detektor-Einheit (1D) erfassten Ergebnisses,
**dadurch gekennzeichnet, dass**
wenn die Schätzungs-Einheit (1F) schätzt, dass der Benutzer nicht läuft, die Steuerungs-Einheit (1E) einen längeren Steuerungs-Zyklus für das Antreiben des Stellgliedes (1C) als dann einstellt, wenn die Schätz-Einheit (1F) schätzt, dass der Benutzer läuft.

2. Kniegelenkprothese (1) nach Anspruch 1, wobei die Schätz-Einheit (1F) schätzt, dass der Benutzer nicht läuft, wenn ein Knie-Winkel, berechnet auf Basis des von der Detektor-Einheit (1D) erfassten Ergebnisses, auf oder über einem ersten Schwellenwert liegt.

3. Kniegelenkprothese (1) nach Anspruch 1, wobei die Schätz-Einheit (1F) schätzt, dass der Benutzer nicht läuft, wenn ein Minimalwert eines Oberschenkel-Winkels, eine Oberschenkel-Winkelgeschwindigkeit, ein Knie-Winkel, eine Knie-Winkelgeschwindigkeit und ein Oberschenkel-Winkel, berechnet auf Basis des von der Detektor-Einheit (1D) erfassten Ergebnisses, einem vorgegebenen Schwellenwert entsprechen.

4. Kniegelenkprothese (1) nach Anspruch 1,
wobei eine erste Bedingung so definiert ist, dass ein Neigungswinkel des Unterschenkelteils (1B), berechnet auf Basis des von der Detektor-Einheit (1D) erfassten Ergebnisses, über eine Dauer eines dritten Schwellenwertes oder länger auf oder über einem zweiten Schwellenwert verbleibt,
eine zweite Bedingung so definiert ist, dass ein Oberschenkel-Winkel einen vierten Schwellenwert erreicht oder überschreitet und ein Knie-Winkel einen fünften Schwellenwert erreicht oder überschreitet, und
die Schätz-Einheit (1F) schätzt, dass der Benutzer nicht läuft, wenn die erste oder die zweite Bedingung erfüllt ist.

5. Kniegelenkprothese (1) nach Anspruch 1,
wobei eine dritte Bedingung so definiert ist, dass ein Knie-Winkel, berechnet auf Basis des von der Detektor-Einheit (1D) erfassten Ergebnisses, einen sechsten Schwellenwert unterschreitet,
eine vierte Bedingung so definiert ist, dass ein Vorwärts-Neigungswinkel des Unterschenkelteils (1B) relativ zu einer vertikalen Achse unter einem siebten Schwellenwert liegt oder der Unterschenkelteil (1B) nach hinten geneigt ist,
eine fünfte Bedingung so definiert ist, dass eine Winkelgeschwindigkeit des Unterschenkelteils (1B) an einer Vorwärtsneigungs-Seite relativ zu der vertikalen Achse auf oder unter einem achten Schwellenwert liegt, oder ein Rückwärts-Neigungswinkel des Unterschenkelteils (1B) zunimmt,
eine sechste Bedingung so definiert ist, dass eine Winkelgeschwindigkeit des Unterschenkelteils (1B), der einen Winkel in einer Vorwärts-Rückwärts-Richtung relativ zu der vertikalen Achse bildet, einen neunten Schwellenwert erreicht oder unterschreitet, und
die Schätz-Einheit (1F) schätzt, dass der Benutzer nicht läuft, wenn eine beliebige Bedingung von der dritten bis zu der sechsten Bedingung erfüllt ist.

6. Kniegelenkprothese (1) nach Anspruch 1,
wobei eine siebte Bedingung so definiert ist, dass ein Absolutwert eines Neigungswinkels des Unterschenkelteils (1B) in einer Vorwärts-Rückwärts-Richtung relativ zu einer vertikalen Achse, berechnet auf Basis des von der Detektor-Einheit (1D) erfassten Ergebnisses, einen zehnten Schwellenwert erreicht oder unterschreitet,
eine achte Bedingung so definiert ist, dass ein Absolutwert einer Winkelgeschwindigkeit des Unterschenkelteils (1B), der einen Winkel in der Vorwärts-Rückwärts-Richtung relativ zu der vertikalen Achse bildet, einen elften Schwellenwert erreicht oder unterschreitet,
eine neunte Bedingung so definiert ist, dass ein Knie-Winkel in einen Bereich von einem zwölften bis zu einem dreizehnten Schwellenwert fällt,
eine zehnte Bedingung so definiert ist, dass ein Absolutwert einer Knie-Winkelgeschwindigkeit einen vierzehnten Schwellenwert erreicht oder unterschreitet, und
die Schätz-Einheit (1F) schätzt, dass der Benutzer nicht läuft, wenn alle Bedingungen von der siebten bis zu der zehnten Bedingung erfüllt sind.

7. Kniegelenkprothese (1) nach Anspruch 1,
wobei eine Verarbeitungs-Einheit (1I) so ausgeführt ist, dass sie, basierend auf dem von der Detektor-Einheit (1D) erfassten Ergebnis, Vorgänge durchführt, und ein erster Prozess so definiert ist, dass die Verarbeitungs-Einheit (1I) so gesteuert wird, dass die Verarbeitungs-Einheit (1I) die Vorgänge weniger häufig als dann durchführt, wenn die Schätz-Einheit (1F) schätzt, dass der Benutzer läuft,
ein zweiter Prozess so definiert ist, dass ein Teil einer Steuerung unterbrochen wird, die durchzuführen ist, wenn die Schätz-Einheit (1F) schätzt, dass der Benutzer läuft,
ein dritter Prozess so definiert ist, dass ein längerer Zyklus zum Ermitteln des von der Detektor-Einheit (1D) erfassten Ergebnisses als dann eingestellt wird, wenn die Schätz-Einheit (1F) schätzt, dass der Benutzer läuft, und
die Steuerungs-Einheit (1E) den ersten Prozess, den zweiten Prozess oder/und den dritten Prozess durchführt, wenn die Schätz-Einheit (1F) schätzt, dass der Benutzer nicht läuft.

8. Kniegelenkprothese (1) nach Anspruch 1, die des Weiteren eine Einheit (1K) zum Erfassen eines Batterie-Status umfasst, mit der ein Status einer Batterie (1J) erfasst wird, die vorhanden ist, um der Detektor-Einheit (1D), der Steuerungs-Einheit (1E) und der Schätz-Einheit (1F) Strom zuzuführen,
wobei die Steuerungs-Einheit (1E) auf Basis der von der Schätz-Einheit (1F) durchgeführten Schätzung und des von der Einheit (1K) zum Erfassen eines Batterie-Status erfassten Status der Batterie (1J) einen längeren Steuerungs-Zyklus für das Antreiben des Stellgliedes (1C) einstellt.

9. Kniegelenkprothese (1) nach Anspruch 1,
wobei, wenn ein Knie-Winkel, berechnet auf Basis des von der Detektor-Einheit (1D) erfassten Ergebnisses, innerhalb eines vorgegebenen Bereiches liegt, die Steuerungs-Einheit (1E) einen längeren Steuerungszyklus für das Antreiben des Stellgliedes (1C) als dann einstellt, wenn der Knie-Winkel außerhalb des vorgegebenen Bereiches liegt.

10. Kniegelenkprothese (1) nach Anspruch 9, die des Weiteren umfasst:
ein Streckseiten-Ventil (1G), mit dem Strecken der Kniegelenkprothese (1) eingeschränkt wird, indem Ausfahren des Stellgliedes (1C) eingeschränkt wird; und
ein Beugeseiten-Ventil (1H), mit dem Beugen der Kniegelenkprothese (1) eingeschränkt wird, indem Einfahren des Stellgliedes (1C) eingeschränkt wird;
wobei, wenn der Knie-Winkel auf 140° oder mehr zunimmt, die Steuerungs-Einheit (1E) das Streckseiten-Ventil (1G) vollständig öffnet, so dass das Strecken der Kniegelenkprothese (1) nicht eingeschränkt wird, das Beugeseiten-Ventil (1H) vollständig öffnet, so dass das Beugen der Kniegelenkprothese (1) nicht eingeschränkt wird, und einen längeren Steuerungszyklus für das Antreiben des Stellgliedes (1C) einstellt als vor Zunahme des Knie-Winkels auf 140° oder mehr.

11. Kniegelenkprothese (1) nach Anspruch 9, die des Weiteren umfasst:
ein Streckseiten-Ventil (1G), mit dem Strecken der Kniegelenkprothese (1) eingeschränkt wird, indem Ausfahren des Stellgliedes (1C) eingeschränkt wird; und
ein Beugeseiten-Ventil (1H), mit dem Beugen der Kniegelenkprothese (1) eingeschränkt wird, indem Einfahren des Stellgliedes (1C) eingeschränkt wird;
wobei eine elfte Bedingung so definiert ist, dass ein Minimalwert eines Oberschenkel-Winkels, berechnet auf Basis des von der Detektor-Einheit (1D) erfassten Ergebnisses, einen fünfzehnten Schwellenwert erreicht oder überschreitet,
eine zwölfte Bedingung so definiert ist, dass eine Oberschenkel-Winkelgeschwindigkeit einen sechzehnten Schwellenwert erreicht oder überschreitet,
eine dreizehnte Bedingung so definiert ist, dass der Knie-Winkel einen siebzehnten Schwellenwert erreicht oder überschreitet,
eine vierzehnte Bedingung so definiert ist, dass eine Knie-Winkelgeschwindigkeit einen achtzehnten Schwellenwert erreicht oder überschreitet,
eine fünfzehnte Bedingung so definiert ist, dass ein Oberschenkel-Winkel um einen neunzehnten Schwellenwert oder mehr größer ist als der Minimalwert des Oberschenkel-Winkels und der Oberschenkel-Winkel einen zwanzigsten Schwellenwert erreicht oder unterschreitet,
wobei die Steuerungs-Einheit (1E) die Kniegelenkprothese (1) in einen freien Fahrrad-Modus versetzt, wenn alle Bedingungen von der elften bis zu der fünfzehnten Bedingung erfüllt sind, und
die Steuerungs-Einheit (1E) des Weiteren einen längeren Steuerungszyklus für das Antreiben des Stellgliedes (1C) einstellt als vor Versetzen der Kniegelenkprothese (1) in den freien Fahrrad-Modus, das Streckseiten-Ventil (1G) vollständig öffnet, so dass das Strecken der Kniegelenkprothese (1) nicht eingeschränkt wird, und das Beugeseiten-Ventil (1H) vollständig öffnet, so dass das Beugen der Kniegelenkprothese (1) nicht eingeschränkt wird.

12. Kniegelenkprothese (1) nach Anspruch 11,
wobei eine sechzehnte Bedingung so definiert ist, dass der Knie-Winkel einen einundzwanzigsten Schwellenwert unterschreitet, der unter dem siebzehnten Schwellenwert liegt,
eine siebzehnte Bedingung so definiert ist, dass der Oberschenkel-Winkel einen zweiundzwanzigsten Schwellenwert unterschreitet, der unter dem zwanzigsten Schwellenwert liegt, und
die Steuerungs-Einheit (1E) nach Versetzen der Kniegelenkprothese (1) in den freien Fahrrad-Modus einen kürzeren Steuerungszyklus für das Antreiben des Stellgliedes (1C) als dann einstellt, wenn die sechzehnte oder die siebzehnte Bedingung erfüllt ist und daraufhin die Kniegelenkprothese (1) in den freien Fahrrad-Modus versetzt wird.

13. Kniegelenkprothese (1) nach Anspruch 9, die des Weiteren umfasst:
ein Streckseiten-Ventil (1G), mit dem Strecken der Kniegelenkprothese (1) eingeschränkt wird, indem Ausfahren des Stellgliedes (1C) eingeschränkt wird; und
ein Beugeseiten-Ventil (1H), mit dem Beugen der Kniegelenkprothese (1) eingeschränkt wird, indem Einfahren des Stellgliedes (1C) eingeschränkt wird;
wobei eine achtzehnte Bedingung so definiert ist, dass ein Neigungs-Winkel des Unterschenkelteils (1B) über eine Dauer eines vierundzwanzigsten Schwellenwertes oder länger auf oder über einem dreiundzwanzigsten Schwellenwert verbleibt,
eine neunzehnte Bedingung so definiert ist, dass ein Oberschenkel-Winkel einen fünfundzwanzigsten Schwellenwert erreicht oder überschreitet und der Knie-Winkel einen sechsundzwanzigsten Schwellenwert erreicht oder überschreitet,
eine zwanzigste Bedingung so definiert ist, dass (i) die Bedingung dahingehend, dass der Neigungs-Winkel des Unterschenkelteils (1B) über die Dauer des vierundzwanzigsten Schwellenwertes oder länger auf oder über dem dreiundzwanzigsten Schwellenwert verbleibt, nicht erfüllt ist und (ii) der Oberschenkel-Winkel einen siebenundzwanzigsten Schwellenwert erreicht oder unterschreitet, der unter dem fünfundzwanzigsten Schwellenwert liegt,
eine einundzwanzigste Bedingung so definiert ist, dass der Knie-Winkel einen achtundzwanzigsten Schwellenwert erreicht oder unterschreitet, der unter dem sechsundzwanzigsten Schwellenwert liegt,
wobei die Steuerungs-Einheit (1E) wenigstens eine erste Steuerung durchführt, die aus der ersten Steuerung und einer zweiten Steuerung ausgewählt wird,
die Steuerungs-Einheit (1E) beim Durchführen der ersten Steuerung die Kniegelenkprothese (1) in einen freien Sitz-Modus versetzt, wenn die achtzehnte oder die neunzehnte Bedingung erfüllt ist, und einen längeren Steuerungszyklus für das Antreiben des Stellgliedes (1C) als vor dem Versetzen der Kniegelenkprothese (1) in den sitzfreien Modus einstellt, das Streckseiten-Ventil (1G) vollständig öffnet, so dass das Strecken der Kniegelenkprothese (1) nicht eingeschränkt wird, und das Beugeseiten-Ventil (1H) vollständig öffnet, so dass das Beugen der Kniegelenkprothese (1) nicht eingeschränkt wird, und
die Steuerungs-Einheit (1E) beim Durchführen der zweiten Steuerung nach dem Versetzen der Kniegelenkprothese (1) in den freien Sitz-Modus einen kürzeren Steuerungszyklus für das Antreiben des Stellgliedes (1C) als dann einstellt, wenn die zwanzigste oder die einundzwanzigste Bedingung erfüllt ist und die Kniegelenkprothese (1) daraufhin in den freien Sitz-Modus versetzt wird.

14. Kniegelenkprothese (1) nach Anspruch 9, die des Weiteren umfasst:
ein Streckseiten-Ventil (1G), mit dem Strecken der Kniegelenkprothese (1) eingeschränkt wird, indem Ausfahren des Stellgliedes (1C) eingeschränkt wird; und
ein Beugeseiten-Ventil (1H), mit dem Beugen der Kniegelenkprothese (1) eingeschränkt wird, indem Einfahren des Stellgliedes (1C) eingeschränkt wird;
wobei eine zweiundzwanzigste Bedingung so definiert ist, dass der Knie-Winkel einen neunundzwanzigsten Schwellenwert unterschreitet,
eine dreiundzwanzigste Bedingung so definiert ist, dass ein Vorwärts-Neigungswinkel des Unterschenkelteils (1B) relativ zu einer vertikalen Achse unter einem dreißigsten Schwellenwert liegt oder der Unterschenkelteil (1B) nach hinten geneigt ist,
eine vierundzwanzigste Bedingung so definiert ist, dass eine Winkelgeschwindigkeit des Unterschenkelteils (1B) an einer Vorwärtsneigungs-Seite relativ zu der vertikalen Achse auf oder unter einem einunddreißigsten Schwellenwert liegt, oder ein Rückwärts-Neigungswinkel des Unterschenkelteils (1B) zunimmt,
eine fünfundzwanzigste Bedingung so definiert ist, dass eine Winkelgeschwindigkeit des Unterschenkelteils (1B), der einen Winkel in einer Vorwärts-Rückwärts-Richtung relativ zu der vertikalen Achse bildet, einen zweiunddreißigsten Schwellenwert erreicht oder unterschreitet,
eine sechsundzwanzigste Bedingung so definiert ist, dass der Knie-Winkel den neunundzwanzigsten Schwellenwert erreicht oder überschreitet,
eine siebenundzwanzigste Bedingung so definiert ist, dass ein Vorwärtsneigungs-Winkel des Unterschenkelteils (1B) relativ zu der vertikalen Achse einen dreiunddreißigsten Schwellenwert erreicht oder überschreitet, der über dem dreißigsten Schwellenwert liegt,
eine achtundzwanzigste Bedingung so definiert ist, dass eine Winkelgeschwindigkeit des Unterschenkelteils (1B) an einer Vorwärtsneigungs-Seite relativ zu der vertikalen Achse einen vierunddreißigsten Schwellenwert erreicht oder überschreitet, oder ein Absolutwert einer Winkelgeschwindigkeit des Unterschenkelteils (1B) in der Vorwärts-Rückwärts-Richtung relativ zu der vertikalen Achse einen fünfunddreißigsten Schwellenwert erreicht oder überschreitet,
wobei die Steuerungs-Einheit (1E) wenigstens eine dritte Steuerung durchführt, die aus der dritten Steuerung und einer vierten Steuerung ausgewählt wird,
die Steuerungs-Einheit (1E) beim Durchführen der dritten Steuerung die Kniegelenkprothese (1) in einen Stillstand-Modus versetzt, wenn eine beliebige der Bedingungen von der zweiundzwanzigsten bis zu der fünfundzwanzigsten Bedingung erfüllt ist, und einen längeren Steuerungszyklus für das Antreiben des Stellgliedes (1C) einstellt als vor dem Versetzen der Kniegelenkprothese (1) in den Stillstand-Modus und das Streckseiten-Ventil (1G) in einen voreingestellten Zustand zwischen einem vollständig geöffneten Zustand und einem vollständig geschlossenen Zustand versetzt und das Beugeseiten-Ventil (1H) in einen nachgebenden Zustand zwischen dem vollständig geöffneten Zustand und dem vollständig geschlossenen Zustand versetzt, und
die Steuerungs-Einheit (1E) beim Durchführen der vierten Steuerung nach dem Versetzen der Kniegelenkprothese (1) in den Stillstand-Modus einen kürzeren Steuerungszyklus für das Antreiben des Stellgliedes (1C) als dann einstellt, wenn alle Bedingungen von der sechsundzwanzigsten bis zu der achtundzwanzigsten Bedingung erfüllt sind und daraufhin die Kniegelenkprothese (1) in den Stillstand-Modus versetzt wird.

15. Kniegelenkprothese (1) nach Anspruch 9, die des Weiteren umfasst:
ein Streckseiten-Ventil (1G), mit dem Strecken der Kniegelenkprothese (1) eingeschränkt wird, indem Ausfahren des Stellgliedes (1C) eingeschränkt wird; und
ein Beugeseiten-Ventil (1H), mit dem Beugen der Kniegelenkprothese (1) eingeschränkt wird, indem Einfahren des Stellgliedes (1C) eingeschränkt wird;
wobei eine neunundzwanzigste Bedingung so definiert ist, dass ein Absolutwert eines Neigungs-Winkels des Unterschenkelteils (1B) in einer Vorwärts-Rückwärts-Richtung relativ zu einer vertikalen Achse auf oder unter einem sechsunddreißigsten Schwellenwert liegt,
eine dreißigste Bedingung so definiert ist, dass ein Absolutwert einer Winkelgeschwindigkeit des Unterschenkelteils (1B), der einen Winkel in der Vorwärts-Rückwärts-Richtung relativ zu der vertikalen Achse bildet, einen siebenunddreißigsten Schwellenwert erreicht oder unterschreitet,
eine einunddreißigste Bedingung so definiert ist, dass der Knie-Winkel in einen Bereich von einem achtunddreißigsten bis zu einem neununddreißigsten Schwellenwert fällt,
eine zweiunddreißigste Bedingung so definiert ist, dass ein Absolutwert einer Knie-Winkelgeschwindigkeit einen vierzigsten Schwellenwert erreicht oder unterschreitet,
eine dreiunddreißigste Bedingung so definiert ist, dass der Absolutwert des Neigungs-Winkels des Unterschenkelteils (1B) in der Vorwärts-Rückwärts-Richtung relativ zu der vertikalen Achse den sechsunddreißigsten Schwellenwert überschreitet,
eine vierunddreißigste Bedingung so definiert ist, dass ein Absolutwert einer Winkelgeschwindigkeit des Unterschenkelteils (1B), der einen Winkel in der Vorwärts-Rückwärts-Richtung relativ zu der vertikalen Achse bildet, den siebenunddreißigsten Schwellenwert überschreitet,
eine fünfunddreißigste Bedingung so definiert ist, dass der Knie-Winkel den achtunddreißigsten Schwellenwert unterschreitet,
eine sechsunddreißigste Bedingung so definiert ist, dass der Knie-Winkel den neununddreißigsten Schwellenwert überschreitet, oder ein Absolutwert der Knie-Winkelgeschwindigkeit den vierzigsten Schwellenwert überschreitet,
wobei die Steuerungs-Einheit (1E) wenigstens eine fünfte Steuerung durchführt, die aus der fünften Steuerung und einer sechsten Steuerung ausgewählt wird,
die Steuerungs-Einheit (1E) beim Durchführen der fünften Steuerung die Kniegelenkprothese (1) in einen Sicherheits-Sperrmodus versetzt, wenn alle Bedingungen von der neunundzwanzigsten bis zu der zweiunddreißigsten Bedingung erfüllt sind, und einen längeren Steuerungszyklus für das Antreiben des Stellgliedes (1C) einstellt als vor dem Versetzen der Kniegelenkprothese (1) in den Sicherheits-Sperrmodus, das Streckseiten-Ventil (1G) vollständig öffnet, so dass das Strecken der Kniegelenkprothese (1) nicht eingeschränkt wird, und das Beugeseiten-Ventil (1H) vollständig schließt, so dass das Beugen der Kniegelenkprothese (1) eingeschränkt wird, und
die Steuerungs-Einheit (1E) beim Durchführen der sechsten Steuerung nach dem Versetzen der Kniegelenkprothese (1) in den Sicherheits-Sperrmodus einen kürzeren Steuerungszyklus für das Antreiben des Stellgliedes (1C) als dann einstellt, wenn eine beliebige Bedingung von der dreiunddreißigsten bis zu der sechsunddreißigsten Bedingung erfüllt ist und daraufhin die Kniegelenkprothese (1) in den Sicherheits-Sperrmodus versetzt wird.

## Revendications

1. Prothèse d'articulation de genou (1) comprenant :
un raccord de cuisse (1A) raccordable à une douille configurée pour recevoir une cuisse d'un utilisateur ;
une partie inférieure de jambe (1B) couplée au raccord de cuisse (1A) de sorte que la partie inférieure de jambe (1B) soit mobile en rotation autour d'un axe d'un genou ;
un actionneur (1C) couplé au raccord de cuisse (1A) et à la partie inférieure de jambe (1B), l'actionneur (1C) étant configuré pour restreindre ou assister un mouvement du raccord de cuisse (1A) ;
une unité de détecteur (1D) pour obtenir directement ou indirectement des informations sur la manière dont le raccord de cuisse (1A) et la partie inférieure de jambe (1B) sont positionnées relativement ;
une unité de commande (1E) pour commander un entraînement de l'actionneur (1C) selon un résultat détecté par l'unité de détecteur (1D) ; et
une unité d'estimation (1F) pour estimer un état d'un mouvement de l'utilisateur selon le résultat détecté par l'unité de détecteur (1D),
**caractérisée en ce que**, lorsque
l'unité d'estimation (1F) estime que l'utilisateur n'est pas en train de marcher, l'unité de commande (1E) définit un cycle de commande plus long pour l'entraînement de l'actionneur (1 C) que lorsque l'unité d'estimation (1F) estime que l'utilisateur est en train de marcher.

2. La prothèse d'articulation de genou (1) de la revendication 1, dans laquelle l'unité d'estimation (1F) estime que l'utilisateur n'est pas en train de marcher lorsqu'un angle de genou calculé selon le résultat détecté par l'unité de détecteur (1D) est supérieur ou égal à une première valeur de seuil.

3. La prothèse d'articulation de genou (1) de la revendication 1, dans laquelle l'unité d'estimation (1F) estime que l'utilisateur n'est pas en train de marcher lorsqu'une valeur minimale d'un angle de cuisse, d'une vitesse angulaire de cuisse, d'un angle de genou, d'une vitesse angulaire de genou, et d'un angle de cuisse calculé selon le résultat détecté par l'unité de détecteur (1D) satisfont une valeur de seuil prédéterminée.

4. La prothèse d'articulation de genou (1) de la revendication 1,
dans laquelle une première condition est définie telle qu'un angle d'inclinaison de la partie inférieure de jambe (1B) calculé selon le résultat détecté par l'unité de détecteur (1D) reste supérieur ou égal à une deuxième valeur de seuil ou plus sur une durée d'une troisième valeur de seuil ou plus,
dans laquelle une deuxième condition est définie de sorte qu'un angle de cuisse devienne supérieur ou égal à une quatrième valeur de seuil et un angle de genou devienne supérieur ou égal à une cinquième valeur de seuil, et
dans laquelle l'unité d'estimation (1F) estime que l'utilisateur n'est pas en train de marcher si la première ou la deuxième condition est satisfaite.

5. La prothèse d'articulation de genou (1) de la revendication 1,
dans laquelle une troisième condition est définie de sorte qu'un angle de genou calculé selon le résultat détecté par l'unité de détecteur (1D) devienne inférieur à une sixième valeur de seuil,
dans laquelle une quatrième condition est définie de sorte qu'un angle d'inclinaison vers l'avant de la partie inférieure de jambe (1B) par rapport à un axe vertical est inférieur à une septième valeur de seuil ou la partie inférieure de jambe (1B) est inclinée vers l'arrière,
dans laquelle une cinquième condition est définie de sorte qu'une vitesse angulaire de la partie inférieure de jambe (1B) sur un côté d'inclinaison vers l'avant par rapport à l'axe vertical est inférieure ou égale à une huitième valeur de seuil, ou un angle d'inclinaison vers l'arrière de la partie inférieure de jambe (1B) diminue,
dans laquelle une sixième condition est définie de sorte qu'une vitesse angulaire de la partie inférieure de jambe (1B) constituant un angle dans une direction avant-arrière par rapport à l'axe vertical devienne inférieure ou égale à une neuvième valeur de seuil, et
dans laquelle l'unité d'estimation (1F) estime que l'utilisateur n'est pas en train de marcher si une quelconque de la troisième à la sixième conditions est satisfaite.

6. La prothèse d'articulation de genou (1) de la revendication 1,
dans laquelle une septième condition est définie de sorte qu'une valeur absolue d'un angle d'inclinaison de la partie inférieure de jambe (1B) dans une direction avant-arrière par rapport à un axe vertical calculé selon le résultat détecté par l'unité de détecteur (1D) devienne inférieure ou égale à une dixième valeur de seuil,
dans laquelle une huitième condition est définie de sorte qu'une valeur absolue d'une vitesse angulaire de la partie inférieure de jambe (1B) constituant un angle dans la direction avant-arrière par rapport à l'axe vertical devienne inférieure ou égale à une onzième valeur de seuil,
dans laquelle une neuvième condition est définie de sorte qu'un angle de genou s'inscrive dans une plage d'une douzième valeur de seuil à une treizième valeur de seuil,
dans laquelle une dixième condition est définie de sorte qu'une valeur absolue d'une vitesse angulaire de genou devienne inférieure ou égale à une quatorzième valeur de seuil, et
dans laquelle l'unité d'estimation (1F) estime que l'utilisateur n'est pas en train de marcher si toutes de la septième à la dixième conditions sont satisfaites.

7. La prothèse d'articulation de genou (1) de la revendication 1,
dans laquelle une unité de traitement (1I) est configurée pour exécuter des opérations selon le résultat détecté par l'unité de détecteur (1D), et un premier processus est défini en tant que commande de l'unité de traitement (1I) de sorte que l'unité de traitement (1I) exécute les opérations moins fréquemment que lorsque l'unité d'estimation (1F) estime que l'utilisateur est en train de marcher,
dans laquelle un deuxième processus est défini en tant qu'interruption d'une partie d'une commande qui est à exécuter lorsque l'unité d'estimation (1F) estime que l'utilisateur est en train de marcher,
dans laquelle un troisième processus est défini en tant que définition d'un cycle plus long pour obtenir le résultat détecté par l'unité de détecteur (1D) que lorsque l'unité d'estimation (1F) estime que l'utilisateur est en train de marcher, et
dans laquelle l'unité de commande (1E) exécute au moins un du premier processus, du deuxième processus ou du troisième processus lorsque l'unité d'estimation (1F) estime que l'utilisateur n'est pas en train de marcher.

8. La prothèse d'articulation de genou (1) de la revendication 1, comprenant en outre une unité d'acquisition d'état de batterie (1K) pour acquérir un état d'une batterie (1J) disposée pour alimenter en énergie l'unité de détecteur (1D), l'unité de commande (1E) et l'unité d'estimation (1F),
dans laquelle l'unité de commande (1E) définit un cycle de commande plus long pour l'entraînement de l'actionneur (1C) selon l'estimation effectuée par l'unité d'estimation (1F) et l'état de la batterie (1J) acquis par l'unité d'acquisition d'état de batterie (1K) .

9. La prothèse d'articulation de genou (1) de la revendication 1,
dans laquelle, lorsqu'un angle de genou calculé selon le résultat détecté par l'unité de détecteur (1D) s'inscrit dans une plage prédéterminée, l'unité de commande (1E) définit un cycle de commande plus long pour l'entraînement de l'actionneur (1C) que lorsque l'angle de genou est hors de la plage prédéterminée.

10. La prothèse d'articulation de genou (1) de la revendication 9, comprenant en outre :
un clapet côté allongement (1G) pour restreindre un allongement de la prothèse d'articulation de genou (1) en restreignant une extension de l'actionneur (1C) ; et
un clapet côté flexion (1H) pour restreindre une flexion de la prothèse d'articulation de genou (1) en restreignant une contraction de l'actionneur (1C),
dans laquelle, lorsque l'angle de genou atteint 140 degrés ou plus, l'unité de commande (1E) ouvre entièrement le clape côté allongement (1G) de sorte que l'allongement de la prothèse d'articulation de genou (1) ne soit pas restreint, ouvre entièrement le clapet côté flexion (1H) de sorte que la flexion de la prothèse d'articulation de genou (1) ne soit pas restreinte, et définit un cycle de commande plus long pour l'entraînement de l'actionneur (1C) qu'avant que l'angle de genou n'atteigne 140 degrés ou plus.

11. La prothèse d'articulation de genou (1) de la revendication 9, comprenant en outre :
un clapet côté allongement (1G) pour restreindre un allongement de la prothèse d'articulation de genou (1) en restreignant une extension de l'actionneur (1C) ; et
un clapet côté flexion (1H) pour restreindre une flexion de la prothèse d'articulation de genou (1) en restreignant une contraction de l'actionneur (1C),
dans laquelle une onzième condition est définie de sorte qu'une valeur minimale d'angle de cuisse calculé selon le résultat détecté par l'unité de détecteur (1D) devienne supérieure ou égale à une quinzième valeur de seuil,
dans laquelle une douzième condition est définie de sorte qu'une vitesse angulaire de cuisse devienne supérieure ou égale à une seizième valeur de seuil,
dans laquelle une treizième condition est définie de sorte que l'angle de genou devienne supérieur ou égal à une dix-septième valeur de seuil,
dans laquelle une quatorzième condition est définie de sorte qu'une vitesse angulaire de genou devienne supérieure ou égale à une dix-huitième valeur de seuil,
dans laquelle une quinzième condition est définie de sorte qu'un angle de cuisse soit supérieur à la valeur minimale de l'angle de cuisse d'une dixième-neuvième valeur de seuil ou plus et que l'angle de cuisse devienne inférieur ou égal à une vingtième valeur de seuil,
dans laquelle l'unité de commande (1E) place la prothèse d'articulation de genou (1) dans un mode libre de vélo lorsque toutes de la onzième à la quinzième conditions sont satisfaites, et
dans laquelle l'unité de commande (1E) définit en outre un cycle de commande plus long pour l'entraînement de l'actionneur (1C) qu'avant que la prothèse d'articulation de genou (1) soit placée dans le mode libre de vélo, ouvre entièrement le clape côté allongement (1G) de sorte que l'allongement de la prothèse d'articulation de genou (1) ne soit pas restreint, et ouvre entièrement le clapet côté flexion (1H) de sorte que la flexion de la prothèse d'articulation de genou (1) ne soit pas restreinte.

12. La prothèse d'articulation de genou (1) de la revendication 11,
dans laquelle une seizième condition est définie de sorte que l'angle de genou devienne inférieur à une vingt-et-unième valeur de seuil qui est inférieure à la dix-septième valeur de seuil,
dans laquelle une dix-septième condition est définie de sorte que l'angle de cuisse devienne inférieur à une vingt-deuxième valeur de seuil qui est inférieure à la vingtième valeur de seuil, et
dans laquelle, après que la prothèse d'articulation de genou (1) est placée dans le mode libre de vélo, l'unité de commande (1E) définit un cycle de commande plus court pour l'entraînement de l'actionneur (1C) que lorsque la prothèse d'articulation de genou (1) est placée dans le mode libre de vélo sur satisfaction de la seizième ou dix-septième condition.

13. La prothèse d'articulation de genou (1) de la revendication 9, comprenant en outre :
un clapet côté allongement (1G) pour restreindre un allongement de la prothèse d'articulation de genou (1) en restreignant une extension de l'actionneur (1C) ; et
un clapet côté flexion (1H) pour restreindre une flexion de la prothèse d'articulation de genou (1) en restreignant une contraction de l'actionneur (1C),
dans laquelle une dix-huitième condition est définie telle qu'un angle d'inclinaison de la partie inférieure de jambe (1B) reste supérieur ou égal à une vingt-troisième valeur de seuil sur une durée d'une vingt-quatrième valeur de seuil ou plus,
dans laquelle une dix-neuvième condition est définie de sorte qu'un angle de cuisse devienne supérieur ou égal à une vingt-cinquième valeur de seuil et l'angle de genou devienne supérieur ou égal à une vingt-sixième valeur de seuil,
dans laquelle une vingtième condition est définie de sorte que (i) la condition que l'angle d'inclinaison de la partie inférieure de jambe (1B) reste supérieur ou égal à une vingt-troisième valeur de seuil sur une durée de la vingt-quatrième valeur de seuil ou plus n'est pas satisfaite et (ii) l'angle de cuisse devienne inférieur ou égal à une vingt-septième valeur de seuil qui est inférieure à la vingt-cinquième valeur de seuil,
dans laquelle une vingt-et-unième condition est définie de sorte que l'angle de genou devienne inférieur ou égal à une vingt-huitième valeur de seuil qui est inférieure à la vingt-sixième valeur de seuil,
dans laquelle l'unité de commande (1E) exécute au moins une première commande sélectionnée à partir de la première commande et d'une deuxième commande,
dans laquelle, lors d'une exécution de la première commande, l'unité de commande (1E) place la prothèse d'articulation de genou (1) dans un mode libre d'assise lorsque la dix-huitième ou dix-neuvième condition est satisfaite, et définit un cycle de commande plus long pour l'entraînement de l'actionneur (1C) qu'avant que la prothèse d'articulation de genou (1) soit placée dans le mode libre d'assise, ouvre totalement le clapet côté allongement (1G) de sorte que l'allongement de la prothèse d'articulation de genou (1) ne soit pas restreint, et ouvre totalement le clapet côté flexion (1H) de sorte que la flexion de la prothèse d'articulation de genou (1) ne soit pas restreinte, et
dans laquelle, lors d'une exécution de la deuxième commande, l'unité de commande (1E) définit, après que la prothèse d'articulation de genou (1) est placée dans le mode libre d'assise, un cycle de commande plus court pour l'entraînement de l'actionneur (1C) que lorsque la prothèse d'articulation de genou (1) est placée dans le mode libre d'assise sur satisfaction de la vingtième ou vingt-et-unième condition.

14. La prothèse d'articulation de genou (1) de la revendication 9, comprenant en outre :
un clapet côté allongement (1G) pour restreindre un allongement de la prothèse d'articulation de genou (1) en restreignant une extension de l'actionneur (1C) ; et
un clapet côté flexion (1H) pour restreindre une flexion de la prothèse d'articulation de genou (1) en restreignant une contraction de l'actionneur (1C),
dans laquelle une vingt-deuxième condition est définie de sorte que l'angle devienne inférieur à une vingt-neuvième valeur de seuil,
dans laquelle une vingt-troisième condition est définie de sorte qu'un angle d'inclinaison vers l'avant de la partie inférieure de jambe (1B) par rapport à un axe vertical est inférieur à une trentième valeur de seuil ou la partie inférieure de jambe (1B) est inclinée vers l'arrière,
dans laquelle une vingt-quatrième condition est définie de sorte qu'une vitesse angulaire de la partie inférieure de jambe (1B) sur un côté d'inclinaison vers l'avant par rapport à l'axe vertical est inférieure ou égale à une trente-et-unième valeur de seuil, ou un angle d'inclinaison vers l'arrière de la partie inférieure de jambe (1B) augmente,
dans laquelle une vingt-cinquième condition est définie de sorte qu'une vitesse angulaire de la partie inférieure de jambe (1B) constituant un angle dans une direction avant-arrière par rapport à l'axe vertical devienne inférieure ou égale à une trente-deuxième valeur de seuil,
dans laquelle une vingt-sixième condition est définie de sorte que l'angle de genou devienne supérieur ou égal à la vingt-neuvième valeur de seuil,
dans laquelle une vingt-septième condition est définie de sorte qu'un angle d'inclinaison vers l'avant de la partie inférieure de jambe (1B) par rapport à l'axe vertical devient supérieur ou égal à une trente-troisième valeur de seuil qui est supérieure à la trentième valeur de seuil,
dans laquelle une vingt-huitième condition est définie de sorte qu'une vitesse angulaire de la partie inférieure de jambe (1B) sur un côté d'inclinaison vers l'avant par rapport à l'axe vertical devienne supérieure ou égale à une trente-quatrième valeur de seuil, ou une valeur absolue d'une vitesse angulaire de la partie inférieure de jambe (1B) dans la direction avant-arrière par rapport à l'axe vertical devienne supérieure ou égale à une trente-cinquième valeur de seuil,
dans laquelle l'unité de commande (1E) exécute au moins une troisième commande sélectionnée à partir de la troisième commande et d'une quatrième commande,
dans laquelle, lors d'une exécution de la troisième commande, l'unité de commande (1E) place la prothèse d'articulation de genou (1) dans un mode immobile debout lorsqu'une quelconque des vingt-deuxième à vingt-cinquième conditions est satisfaite, et définit un cycle de commande plus long pour l'entraînement de l'actionneur (1C) qu'avant que la prothèse d'articulation du genou (1) soit placée dans le mode immobile debout et place le clapet côté allongement (1G) dans un état pré-ajusté entre un état totalement ouvert et un état totalement fermé et place le clapet côté flexion (1H) dans un état de fonctionnement entre l'état totalement ouvert et l'état totalement fermé, et
dans laquelle, lors d'une exécution de la quatrième commande, l'unité de commande (1E) définit, après que la prothèse d'articulation de genou (1) est placée dans le mode immobile debout, un cycle de commande plus court pour l'entraînement de l'actionneur (1C) que lorsque la prothèse d'articulation de genou (1) est placée dans le mode immobile debout sur satisfaction de toutes des vingt-sixième à vingt-huitième conditions.

15. La prothèse d'articulation de genou (1) de la revendication 9, comprenant en outre :
un clapet côté allongement (1G) pour restreindre un allongement de la prothèse d'articulation de genou (1) en restreignant une extension de l'actionneur (1C) ; et
un clapet côté flexion (1H) pour restreindre une flexion de la prothèse d'articulation de genou (1) en restreignant une contraction de l'actionneur (1C),
dans laquelle une vingt-neuvième condition est définie de sorte qu'une valeur absolue d'un angle d'inclinaison de la partie inférieure de jambe (1B) dans une direction avant-arrière par rapport à un axe vertical soit inférieure ou égale à un trente-sixième seuil,
dans laquelle une trentième condition est définie de sorte qu'une valeur absolue d'une vitesse angulaire de la partie inférieure de jambe (1B) constituant un angle dans la direction avant-arrière par rapport à l'axe vertical devienne inférieure ou égale à une trente-septième valeur de seuil,
dans laquelle une trente-et-unième condition est définie de sorte qu'un angle de genou s'inscrive dans une plage d'une trente-huitième valeur de seuil à une trente-neuvième valeur de seuil,
dans laquelle une trente-deuxième condition est définie de sorte qu'une valeur absolue d'une vitesse angulaire de genou devienne inférieure ou égale à une quarantième valeur de seuil,
dans laquelle une trente-troisième condition est définie de sorte que la valeur absolue de l'angle d'inclinaison de la partie inférieure de jambe (1B) dans la direction avant-arrière par rapport à l'axe vertical devienne supérieure à une trente-sixième valeur de seuil,
dans laquelle une trente-quatrième condition est définie de sorte qu'une valeur absolue d'une vitesse angulaire de la partie inférieure de jambe (1B) constituant un angle dans la direction avant-arrière par rapport à l'axe vertical devienne supérieure à la trente-septième valeur de seuil,
dans laquelle une trente-cinquième condition est définie de sorte que l'angle de genou devienne inférieur à la trente-huitième valeur de seuil,
dans laquelle une trente-sixième condition est définie de sorte que l'angle de genou devienne supérieur à la trente-neuvième valeur de seuil ou une valeur absolue de la vitesse angulaire de genou devienne supérieure à la quarantième valeur de seuil,
dans laquelle l'unité de commande (1E) exécute au moins une cinquième commande sélectionnée à partir de la cinquième commande et d'une sixième commande,
dans laquelle, lors d'une exécution de la cinquième commande, l'unité de commande (1E) place la prothèse d'articulation de genou (1) dans un mode de verrou de sécurité lorsque toutes des vingt-neuvième à trente-deuxième conditions sont satisfaites, et définit un cycle de commande plus long pour l'entraînement de l'actionneur (1C) qu'avant que la prothèse d'articulation de genou (1) soit placée dans le mode de verrou de sécurité, ouvre totalement le clapet côté allongement (1G) de sorte que l'allongement de la prothèse d'articulation de genou (1) ne soit pas restreint, et ferme totalement le clapet côté flexion (1H) de sorte que la flexion de la prothèse d'articulation de genou (1) soit restreinte, et
dans laquelle, lors d'une exécution de la sixième commande, l'unité de commande (1E) définit, après que la prothèse d'articulation de genou (1) est placée dans le mode de verrou de sécurité, un cycle de commande plus court pour l'entraînement de l'actionneur (1C) que lorsque la prothèse d'articulation de genou (1) est placée dans le mode de verrou de sécurité sur satisfaction d'une quelconque des trente-troisième à trente-sixième conditions.
